(19) Europäisches Patentamt
European Patent Office
Office européen des brevets

(11) EP 0 663 399 B1

(12) **EUROPÄISCHE PATENTSCHRIFT**

(45) Veröffentlichungstag und Bekanntmachung des Hinweises auf die Patenterteilung:
**10.06.1998 Patentblatt 1998/24**

(21) Anmeldenummer: **94119742.8**

(22) Anmeldetag: **14.12.1994**

(51) Int Cl.[6]: **C07D 471/04**, C07D 491/04, C07D 213/80, A01N 43/40 // (C07D471/04, 221:00, 209:00), (C07D491/04, 307:00, 221:00)

(54) **Pyridin-2,3-dicarbonsäureimide, Verfahren zu ihrer Herstellung und ihre Verwendung zur Bekämpfung unerwünschten Pflanzenwuchses**

Pyridine-2,3-dicarboxylic imides, process for their preparation and their use to control the growth of unwanted plants

Pyridine-2,3-dicarboxylique imides, procédé pour leur préparation et leur utilisation pour combattre la croissance de plantes indésirables

(84) Benannte Vertragsstaaten:
**BE CH DE FR GB IT LI NL**

(30) Priorität: **22.12.1993 DE 4343922**

(43) Veröffentlichungstag der Anmeldung:
**19.07.1995 Patentblatt 1995/29**

(73) Patentinhaber: **BASF AKTIENGESELLSCHAFT**
**67056 Ludwigshafen (DE)**

(72) Erfinder:
- **Hamprecht, Dr. Gerhard**
  **D-69469 Weinheim (DE)**
- **Münster, Dr. Peter**
  **D-68809 Neulussheim (DE)**
- **Gerber, Dr. Matthias**
  **D-67117 Limburgerhof (DE)**
- **Walter, Dr. Helmuth**
  **D-67283 Obrigheim (DE)**
- **Westphalen, Dr. Karl-Otto**
  **D-67346 Speyer (DE)**

(56) Entgegenhaltungen:
**EP-A- 0 041 623**
**EP-A- 0 128 006**
**EP-A- 0 322 616**
**EP-A- 0 422 456**
**GB-A- 2 174 395**

- **CHEMICAL ABSTRACTS, vol. 111, no. 13, 25. September 1989, Columbus, Ohio, US; abstract no. 112292p,**
- **CHEMICAL ABSTRACTS, vol. 81, no. 25, 23. Dezember 1974, Columbus, Ohio, US; abstract no. 169499c,**
- **CHEMICAL ABSTRACTS, vol. 80, no. 19, 13. Mai 1974, Columbus, Ohio, US; abstract no. 108324z,**
- **JOURNAL OF HETEROCYCLIC CHEMISTRY, Bd.30, Nr.1, 1993, PROVO US Seiten 295 - 299 Y. TOMINAGA**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die vorliegende Erfindung betrifft Pyridin-2,3-dicarbonsäureimide der allgemeinen Formel I

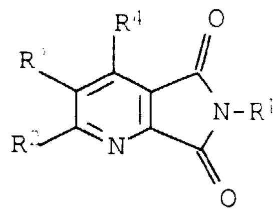

I

in der

$R^1$

Wasserstoff;

$C_1$-$C_4$-Alkoxy;

$C_1$-$C_6$-Alkyl, das eine bis drei der folgenden Gruppen tragen kann: $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Dialkylamino, $C_3$-$C_8$-Cycloalkyl, Halogen, ausgenommen $C_1$-$C_4$-Dialkylaminoethyl, wenn einer der Reste $R^2$, $R^3$ oder $R^4$ Amino oder Hydroxy bedeutet;

$C_3$-$C_8$-Cycloalkyl, das eine bis drei der folgenden Gruppen tragen kann: $C_1$-$C_6$-Alkyl, $C_1$-$C_6$-Halogenalkyl, $C_1$-$C_4$-Alkoxy, $C_1$-$C_4$-Halogenalkoxy, Halogen oder Nitro;

$C_3$-$C_6$-Alkenyl oder $C_3$-$C_6$-Alkinyl, die bis zu dreimal durch Halogen substituiert sein können, bedeutet und mindestens ein Rest $R^2$, $R^3$ oder $R^4$ für eine Gruppe $NR^6R^7$ steht und die übrigen Reste $R^2$, $R^3$ bzw. $R^4$ wie folgt definiert sind:

i) Wasserstoff;

ii) Halogen oder Cyano;

iii) $C_1$-$C_6$-Alkyl, welches durch ein bis fünf Halogenatome und/oder einen bis zwei der folgenden Reste substituiert sein kann: $C_1$-$C_4$-Alkoxy, $C_1$-$C_4$-Halogenalkoxy, $C_1$-$C_4$-Alkylthio, $C_1$-$C_4$-Halogenalkylthio, $C_3$-$C_6$-Cycloalkyl oder Cyano;

iv) Benzyl, das bis zu dreimal durch $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro substituiert sein kann;

v) $C_3$-$C_8$-Cycloalkyl, das ein- bis dreimal durch $C_1$-$C_4$-Alkyl oder Halogen substituiert sein kann;

vi) $C_2$-$C_6$-Alkenyl, welches bis zu dreimal durch Halogen und/oder einmal durch $C_1$-$C_3$-Alkoxy oder durch Phenyl, das eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro, substituiert sein kann;

vii) $C_2$-$C_6$-Alkinyl, welches bis zu dreimal durch Halogen oder $C_1$-$C_3$-Alkoxy und/oder einmal durch Phenyl, das eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro, substituiert sein kann;

viii) $C_1$-$C_4$-Alkoxy, $C_1$-$C_4$-Alkylthio, $C_1$-$C_4$-Halogenalkoxy, $C_1$-$C_4$-Halogenalkylthio, $C_2$-$C_5$-Alkenyloxy, $C_2$-$C_5$-Alkinyloxy, $C_1$-$C_4$-Alkylsulfinyl, $C_1$-$C_4$-Alkylsulfonyl, $C_1$-$C_4$-Halogenalkylsulfonyl;

ix) Phenoxy oder Phenylthio, welche bis zu dreimal durch $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro substituiert sein können;

x) ein 5- oder 6-gliedriger heterocyclischer Rest mit einem oder zwei Heteroatomen, ausgewählt aus der Gruppe Sauerstoff, Schwefel und Stickstoff, der ein bis zwei Substituenten der folgenden Gruppen tragen kann: $C_1$-$C_3$-Alkyl, Halogen, $C_1$-$C_3$-Alkoxy oder $C_2$-$C_4$-Alkoxycarbonyl;

xi) Phenyl, welches eine bis drei der folgenden Gruppen tragen kann: $C_1$-$C_6$-Alkyl, $C_1$-$C_6$-Halogenalkyl, $C_1$-$C_6$-

Alkoxy, $C_1$-$C_6$-Halogenalkoxy, $C_1$-$C_6$-Alkylthio, $C_1$-$C_6$-Halogenalkylthio, Halogen, Nitro oder Cyano;

xii) eine Gruppe $OR^5$, wobei $R^5$ Wasserstoff, $C_1$-$C_4$-Alkylcarbonyl, $C_1$-$C_4$-Halogenalkylcarbonyl, $C_1$-$C_4$-Alkoxycarbonyl, $C_1$-$C_4$-Alkoxy-$C_2$-$C_4$-alkoxycarbonyl, $C_1$-$C_4$-Alkylcarbamoyl, $C_1$-$C_4$-Dialkylcarbamoyl, $C_1$-$C_4$-Alkylsulfonyl, $C_1$-$C_4$-Halogenalkylsulfonyl, Sulfamoyl, $C_1$-$C_4$-Alkylaminosulfonyl, $C_1$-$C_4$-Dialkylaminosulfonyl, Phenylsulfonyl, welches ein- bis dreimal durch $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, Halogen, Cyano oder Nitro substituiert sein kann, bedeutet;

xiii) eine Gruppe $NR^6R^7$, wobei $R^6$ Wasserstoff, $C_1$-$C_4$-Alkyl, Benzyl, $C_1$-$C_4$-Alkoxy oder zusammen mit $R^7$ C=S bedeutet und $R^7$ für Wasserstoff, $C_1$-$C_4$-Alkyl, $C_1$-$C_4$-Alkylcarbonyl, $C_1$-$C_4$-Halogenalkylcarbonyl, $C_1$-$C_4$-Alkoxycarbonyl, $C_1$-$C_4$-Alkoxy-$C_2$-$C_4$-alkoxycarbonyl, $C_1$-$C_4$-Alkylcarbamoyl, $C_1$-$C_4$-Dialkylcarbamoyl, $C_1$-$C_4$-Alkylsulfonyl, $C_1$-$C_4$-Halogenalkylsulfonyl, Sulfamoyl, $C_1$-$C_4$-Alkylaminosulfonyl, $C_1$-$C_4$-Dialkylaminosulfonyl, Phenylsulfonyl, welches ein- bis dreimal durch $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, Halogen, Cyano oder Nitro substituiert sein kann, steht;

ausgenommen 6-Amino-5-cyano-4-phenyl-pyridin-2,3-dicarbonsäureimid;
außerdem kann der Rest $R^3$ unabhängig von der jeweiligen Bedeutung von $R^2$ und $R^4$ die Bedeutung Nitro haben; sowie deren landwirtschaftlich anwendbare Salze.

Weiterhin betrifft die Erfindung Verfahren zur Herstellung der Verbindungen I und ihre Verwendung zur Bekämpfung unerwünschten Pflanzenwuchses. Gegenstand der Erfindung sind ferner neue Pyridin-2,3-dicarbonsäureester sowie Pyridin-2,3-dicarbonsäureanhydride, jeweils insbesondere in 3-Stellung am Pyridinring nitrosubstituiert, die als Zwischenprodukte zur Herstellung der Verbindungen I dienen.

N-substituierte Pyridindicarbonsäureimide bzw. deren Derivate sind bekannt. In EP-A 128 006 werden u.a. N-Cycloalkylen-pyridindicarbonsäureimide und deren Verwendung als Bodenfungizide beschrieben.

In US-A 3 539 568 wird ein Verfahren zur Herstellung von 2,3- und 3,4-Pyridindicarbonsäureimiden und deren Umsetzung zu isomeren Dicarbonsäureamiden beschrieben, die als Zwischenprodukte für herbizide Pyrimidindione verwendet werden können.

Aus US-A 4 261 730 sind 3-Carboxy-pyridin-2-N-(aryl)-carbonsäureamide bekannt sowie Phthalamidsäuren mit wachstumsregulierender Wirkung.

In US-A 4 658 030 wird ein Verfahren zur Herstellung herbizider 2-(Imidazolin-2-yl)-nikotinsäuren auf Basis von 3-Carboxypyridin-2-(N-2-carbamido-3-methyl-2-butyl)-carbonsäureamiden offenbart.

Aus Helv. Chim. Acta 1988, Bd. 71, S. 486 und 493 ist ein Cycloadditions-Verfahren zur Herstellung von Pyridin-2,3-dicarboximiden bekannt.

J 5 7085-386 beschreibt speziell substituierte Pyridin-2,3-dicarbonsäureimide mit Antitumorwirkung. 6-Amino-5-cyano-4-phenylpyridincarbonsäureimid ist aus CA 117, 150 849 bekannt.

Herbizid wirksame Pyridin-2,3-dicarbonsäureimide sind aus EP-A 422 456, EP-A 41 623 und aus GB-A 2 174 395 bekannt.

Zwischenprodukte aus der Gruppe der Pyridin-2,3-dicarbonsäurediestergruppe sind dem Stand der Technik nur vereinzelt zu entnehmen. So beschreibt EP-A 227 932 Pyridin-2,3-dicarbonsäurediester mit folgendem Substitutionsmuster in 5/6-Position: $CH_3/NO_2$, $NH_2/NH_2$, $NO_2/Cl$ und $NO_2/NH_2$. 4-Amino-pyridin-2,3-dicarbonsäuredimethylester ist aus Chemical Abstract 111, 112292p (1989) bekannt, 5-Amino-6-methylpyridin-2,3-dicarbonsäuredimethyloder -diethylester aus Beilstein Nr. 4-22-006875 (Jones, Am. Soc. 74 (1972), S. 1489). 5-Acetamino- und 5-Methylamino-6-methylpyridin-2,3-dicarbonsäurediethylester sind in EP-A 322 616 publiziert. 4-Diethylamino-5-methyl- und 5-Diethylamino-4-methylpyridin werden in Chemical Abstracts 81, 169499c (1974) bzw. 79, 31999t (1973) beschrieben.

Der Erfindung lag nun die Aufgabe zugrunde, Pyridin-2,3-dicarbonsäureimide mit verbesserter biologischer Wirksamkeit insbesondere besserer Selektivität bzw. hoher herbizide Aktivität bereitzustellen.

Demgemäß wurden die eingangs definierten Pyridin-2,3-dicarbonsäureimide gefunden.

Bevorzugt sind Pyridin-2,3-dicarbonsäureimide der Formel I, in der $R^1$ Wasserstoff, $C_1$-$C_6$-Alkyl, $C_3$-$C_6$-Alkenyl, $C_3$-$C_6$-Alkinyl, 1-(Cyclopropyl)-$C_1$-$C_3$-alkyl, 1-$C_1$-$C_2$-Alkyl-cyclopropyl oder $C_3$-$C_6$-Cycloalkyl bedeutet und zwei der drei Reste im Pyridinring $R^2$, $R^3$ oder $R^4$ unabhängig voneinander Wasserstoff, $C_1$-$C_4$-Alkyl, welches durch ein bis fünf Halogenatome substituiert sein kann, $C_1$-$C_4$-Alkoxy, Halogen oder Cyano bedeuten, während der dritte Substituent für eine Gruppe $NR^6R^7$, insbesondere Amino, Methylamino, Dimethylamino, Acetamino, Trifluoracetamino steht.

Besonders bevorzugt steht $R^3$ für Nitro. Ferner sind Verbindungen mit Mono- oder Disubstitution im Pyridinring bevorzugt. Der Substituent am Imidstickstoff $R^1$ stellt vorzugsweise einen Alkylrest dar, insbesondere einen verzweigten Alkylrest wie i-Propyl, sek.-Butyl oder tert.-Butyl dar oder einen 1-(Cycloalkyl)-$C_1$-$C_3$-alkylrest wie 1-(Cyclopropyl)-methyl, 1-(Cyclopropyl)-ethyl oder 1-(Cyclopropyl)-propyl.

Gegenstand der Erfindung sind ferner neue Zwischenprodukte wie Anhydride und substituierte Pyridin-2,3-dicarbonsäureester. Hier sind folgende Verbindungen zu nennen:

Pyridin-2,3-dicarbonsäureanhydride der Formel IIa und IIc

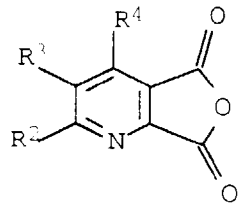

IIa,c

in der mindestens einer der Reste $R^2$, $R^3$ oder $R^4$ $NR^6R^7$ und/oder $R^3$ Nitro bedeutet und die übrigen Reste die eingangs für die Endprodukte I genannten Bedeutungen haben.

Erfindungsgemäße Zwischenprodukte auf der Dialkylesterstufe sind m-Nitro oder Amino substituierte Pyridin-2,3-dicarbonsäure-dialkylester der Formeln Vb und Vd

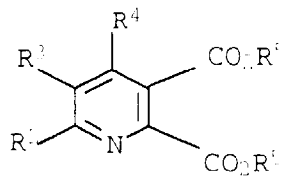

Vb,d

in denen $R^5$ einen $C_1$-$C_4$-Alkyl-, $C_3$-$C_5$-Alkenyl- oder $C_3$-$C_5$-Alkinylrest darstellt, wobei die genannten Reste jeweils durch Halogen, insbesondere ein bis drei Halogenreste wie Fluor, Chlor oder Brom, durch $C_1$-$C_4$-Alkoxy oder durch Phenyl substituiert sein können, und die Reste $R^2$ bis $R^4$ folgende Bedeutung haben:

Vb: $R^3$ steht für eine Nitrogruppe und die Reste $R^2$ und $R^4$ haben die eingangs wie für das Endprodukt I genannte Bedeutung, mit der Maßgabe, daß $R^2$ nicht Methyl, Halogen oder Amino ($NH_2$) darstellt, wenn $R^4$ Wasserstoff bedeutet;

Vd: die Reste $R^2$ , $R^3$ und/oder $R^4$ stehen für eine Gruppe $NR^6R^7$ und die übrigen Reste $R^2$, $R^3$ oder $R^4$ haben die eingangs wie für das Endprodukt I genannte Bedeutung, ausgenommen 5-Amino-6-methyl-pyridin-2,3-dicarbonsäurediethyl- und -dimethylester, 4-Amino-pyridin-2,3-dicarbonsäuredimethylester, 5,6-Diamino-pyridin-2,3-dicarbonsäurediethylester, 6-Amino-5-nitro-pyridin-2,3-dicarbonsäurediethylester, 5-Acetamino-6-methyl-pyridin-2,3-dicarbonsäurediethylester, 5-Methylamino-6-methyl-pyridin-2,3-dicarbonsäurediethylester, 4-Diethylamino-5-methyl-pyridin-2,3-dicarbonsäurediethyl und dimethylester, 6-Diethylamino-5-methylpyridin-2,3-dicarbonsäurediethylester, 5-Acetylamino-pyridin-2,3-dicarbonsäurediethylester, 5-(N-Methyl-N-Acetylamino)-6-methylpyridin-2,3-dicarbonsäurediethylester, 5-Acetylamino-6-methyl-pyridin-2,3-dicarbonsäurediethylester, 4-Amino-5-cyano-6-(4-methylphenyl)sulfonylamino-pyridin-2,3-dicarbonsäuredimethylester.

Die Pyridin-2,3-dicarbonsäureimide der Formel I können mit anorganischen Säuren oder mit Alkylhalogeniden Additionssalze bilden oder sie können, sofern einer der Substituenten saure Eigenschaften hat, mit anorganischen und organischen Basen zu Salzen umgesetzt werden. Die entsprechenden Salze gehören ebenfalls zur Erfindung.

Als basische Salze kommen z.B. diejenigen der Alkalimetalle, vorzugsweise die Natrium- und Kaliumsalze, die der Erdalkalimetalle, vorzugsweise Calcium-, Magnesium-, und Bariumsalze und die der Übergangsmetalle, vorzugsweise Mangan-, Kupfer-, Zink- und Eisensalze sowie die Ammoniumsalze, die ein bis drei $C_1$-$C_4$-Alkyl-, Hydroxy-$C_1$-$C_4$-alkylsubstituenten und/oder einen Phenyl- oder Benzylsubstituenten tragen können, vorzugsweise Diisopropylammonium-, Tetramethylammonium-, Tetrabutylammonium-, Trimethylbenzylammonium- und Trimethyl-(2-hydroxyethyl)-ammoniumsalze, die Phosphoniumsalze, die Sulfoniumsalze, vorzugsweise Tri-($C_1$-$C_4$-)alkylsulfoniumsalze, und die Sulfoxoniumsalze, vorzugsweise Tri-($C_1$-$C_4$-)alkylsulfoxoniumsalze in Betracht.

Sofern das Substitutionsmuster am Pyridin-2,3-dicarbonsäureimid I zu optisch aktiven Verbindungen führt, so gehören zum Gegenstand der Erfindung neben den Racematen auch die (+) und (-) Enantiomeren.

Die Pyridin-2,3-dicarbonsäureimide der Formel I sind auf verschiedenen Wegen herstellbar, die in EP-A 422 456 beschrieben sind.

Erfindungsgemäß erhält man Pyridinderivate der Formel I durch Umsetzung von Pyridindicarbonsäureanhydriden II

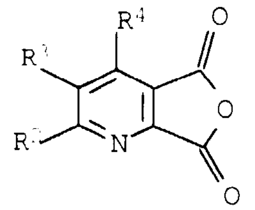

II

in einem inerten organischen Lösungsmittel mit einem primären Amin III (bevorzugt in ungefähr stöchiometrischen Mengen)

$$H_2N\text{-}R^1 \qquad III$$

zu einem Pyridindicarbonsäurehalbamid IV

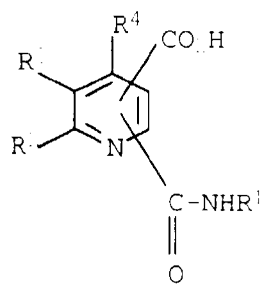

IV

und dessen Cyclisierung mit wasserabspaltenden Mitteln zu I.

Außerdem wurden chemisch eigenartige Verfahren zur Herstellung der Verbindungen Ia gefunden,

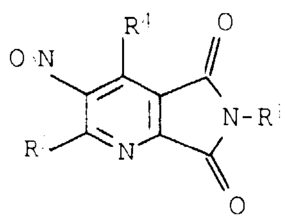

Ia

in denen $R^2$ und $R^4$ die vorgenannte Bedeutung besitzen, indem man Pyridinderivate Ib,

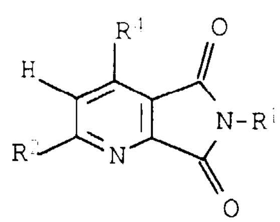

Ib

oder die N-Oxide von Ib mit nitrierenden Agentien behandelt und gegebenenfalls anschließend die N-Oxid-Gruppe entfernt.

Im Rahmen der Herstellung der erfindungsgemäßen Verbindungen wurde weiterhin gefunden, daß man Pyridinderivate der Formel Ia ebenfalls vorteilhaft erhält, wenn man von Pyridindicarbonsäureanhydriden der Formel IIb (oder deren N-Oxiden) ausgeht,

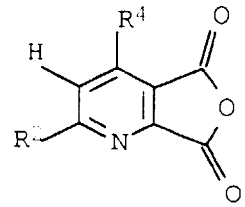

IIb

in der $R^2$ und $R^4$ die vorgenannte Bedeutung besitzen, diese mit nitrierenden Agentien umsetzt zu Pyridindicarbonsäureanhydriden IIa

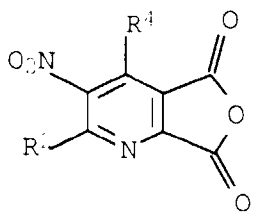

IIa

in denen $R^2$ und $R^4$ die vorgenannte Bedeutung besitzen, und diese dann mit einem substituierten Amin, wie beschrieben, in die Pyridinderivate Ia überführt.

Die Darstellung der Pyridindicarbonsäurehalbamide IV wird zweckmäßigerweise so durchgeführt, daß man das Anhydrid II in einem inerten Lösungsmittel vorlegt und etwa molare Mengen eines Amins III, gegebenenfalls ebenfalls gelöst in einem inerten Lösungsmittel, zutropft. Nach beendeter Reaktion wird das Reaktionsprodukt abgesaugt oder durch Einengen des verwendeten Lösungsmittels isoliert, wobei man die Halbamide IV erhält.

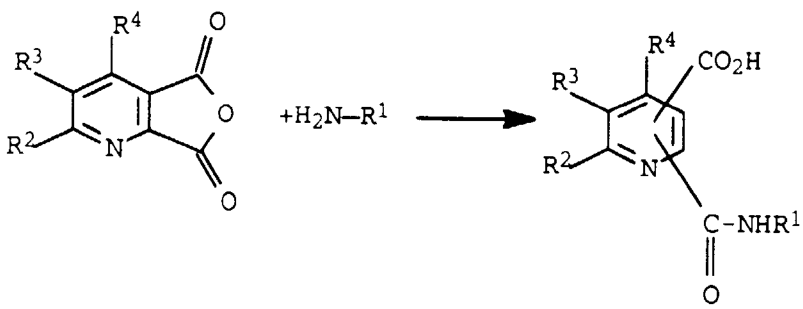

II III IV

Zweckmäßigerweise verwendet man für diese Umsetzungen Lösungsmittel wie Halogenkohlenwasserstoffe, z.B. Tetrachlorethan, Methylenchlorid, Chloroform, Chlorbenzol und 1,2-Dichlorbenzol; Ether z.B. Diethylether, Methyl-tert.-butylether, Dimethoxyethan, Diethylenglykoldimethylether, Tetrahydrofuran und Dioxan; dipolare aprotische Lösungsmittel, z.B. Acetonitril, Dimethylformamid, Dimethylacetamid, Dimethylsulfoxid, N-Methylpyrrolidon, 1,3-Dimethyltetrahydro-2(1H)-pyrimidinon und 1,3-Dimethylimidazolidin-2-on; Aromaten, z.B. Benzol, Toluol, Xylol, Pyridin und Chinolin; Ketone, z.B. Aceton, Methylethylketon oder entsprechende Gemische.

Die Umsetzung kann bei Temperaturen von -10°C bis zur Rückflußtemperatur des jeweiligen Lösungsmittels bzw. -gemisches, vorzugsweise bei -20 bis 120°C, durchgeführt werden.

Die molaren Verhältnisse, in denen die benötigten Ausgangsverbindungen miteinander umgesetzt werden, betragen vorzugsweise 0,9:1 bis 3:1 für das Verhältnis von Amin III zu Anhydrid II. Die Konzentration der Edukte im Lösungsmittel beträgt z.B. 0,1 bis 5 mol/l, bevorzugt 0,2 bis 2 mol/l.

Die für dieses Verfahren als Ausgangsmaterialien benötigten Pyridindicarbonsäuren bzw. -anhydride sind kommerziell erhältlich, literaturbekannt oder können nach allgemein bekannten Methoden hergestellt werden. Eine Übersicht findet sich in Beilstein H 22, 150-160, E I 531-536, E II 104-111, H 27, 261, E I 319, E II 299, R. C. Elderfield, Heterocyclic Compounds, Vol. I, Chapt. 8, J. Wiley and Sons, N.Y., E. Klingberg "Pyridine and its Derivatives", Part 3,

Chapt. X, in The Chemistry of Heterocyclic Compounds, 1962, Interscience Publishers sowie in EP-A 299 362 und EP-A 422 456.

Die Cyclisierung der Halbamide IV erfolgt durch Wasserentzug mit üblichen wasserabspaltenden Mitteln, beispielsweise Acetanhydrid oder anorganischen Säurehalogeniden, wie Thionylchlorid, Phosgen, Phosphortri- oder pentachlorid, zu den Pyridinderivaten der Formel I. Die Reaktion wird zweckmäßigerweise so durchgeführt, daß man die Carbonsäureamide in einem inerten organischen Lösungsmittel vorlegt und das wasserabspaltende Mittel, gegebenenfalls ebenfalls gelöst in einem inerten Lösungsmittel zutropft. Das Gemisch kann wie üblich aufgearbeitet werden, beispielsweise durch Hydrolyse mit Wasser und Absaugen oder Extraktion des Produktes mit einem organischen Lösungsmittel und Einengen des organischen Lösungsmittels:

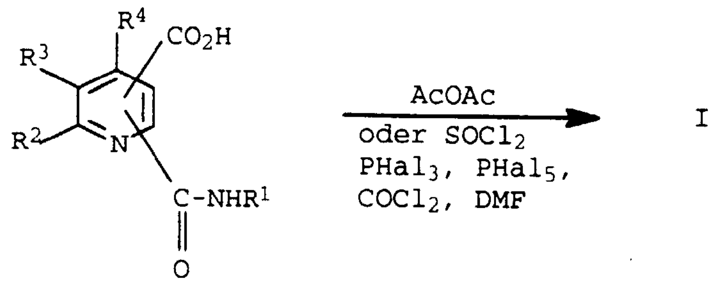


IV

Zweckmäßigerweise verwendet man für diese Umsetzungen Lösungsmittel wie Halogenkohlenwasserstoffe, z.B. Tetrachlorethan, Methylenchlorid, Chloroform, Chlorbenzol und 1,2-Dichlorbenzol; Ether z.B. Diethylether, Methyl-tert.-butylether, Dimethoxyethan, Diethylenglykoldimethylether, Tetrahydrofuran und Dioxan; dipolare aprotische Lösungsmittel, z.B. Acetonitril, Dimethylformamid, Dimethylacetamid, Dimethylsulfoxid, N-Methylpyrrolidon, 1,3-Dimethyltetrahydro-2(1H)-pyrimidinon und 1,3-Dimethylimidazolidin-2-on; Aromaten, z.B. Benzol, Toluol, Xylol, Pyridin und Chinolin; Ketone, z.B. Aceton, Methylethylketon oder entsprechende Gemische.

Die Umsetzung kann bei Temperaturen von -10°C bis zur Rückflußtemperatur des jeweiligen Lösungsmittels, vorzugsweise bei 0 bis 150°C, durchgeführt werden.

Die molaren Verhältnisse, in denen die Ausgangsverbindungen miteinander umgesetzt werden, betragen im allgemeinen 0,9:1 bis 5:1 wasserabspaltendes Mittel zu Säureamid.

Die Konzentration der Edukte im Lösungsmittel(gemisch) beträgt im allgemeinen 0,1 bis 5 mol/l, bevorzugt 0,2 bis 2 mol/l.

Ein Verfahren zur Herstellung von Verbindungen Ia, in denen $R^3$ für Nitro steht, besteht in der Behandlung eines Pyridinderivats Ib mit nitrierenden Agentien.

Die Reaktion wird bevorzugt so durchgeführt, daß man ein Pyridinderivat Ib mit Salpetersäure allein oder in Mischung mit Schwefelsäure - allgemein als Nitriersäure bezeichnet - behandelt und gegebenenfalls wieder entoxygeniert.

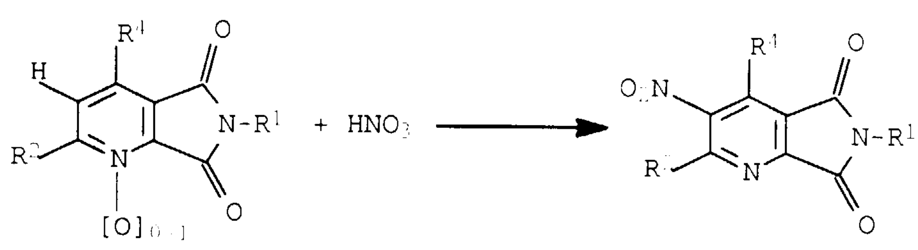

Ib Ia

Zur Ausführung der Nitrierung kann man das Pyridinderivat Ib in Schwefelsäure lösen oder suspendieren und Salpetersäure oder Nitriersäure zugeben. Mitunter ist es von Vorteil das Pyridinderivat Ib in vorgelegte Nitriersäure oder Salpetersäure einzutragen. Die Reaktion kann mit verdünnter Salpetersäure [D: 1,0-1,37] durchgeführt werden,

zweckmäßiger ist jedoch die Verwendung von konzentrierter bis rauchender Salpetersäure [D: 1,37-1,52]. Im Falle der Verwendung von Nitriersäure setzt man üblicherweise Gemische von z.B. 20 % Salpetersäure, 60 % Schwefelsäure, 20 % Wasser bis zu 45 % Salpetersäure und 55 % Schwefelsäure ein.

Eine Steigerung der Nitrierwirkung erfolgt durch Einsatz von Gemischen rauchender Salpetersäure [D: 1,52] mit rauchender Schwefelsäure (Oleum). Anstelle von Schwefelsäure kann man auch Phosphorsäure oder flüssigen Fluorwasserstoff verwenden.

Vorteilhaft kann die Nitrierung auch in Gegenwart von Eisessig durchgeführt werden. Hierzu löst oder suspendiert man das Pyridinderivat Ib in Eisessig und gibt 60 bis 100 %ige Salpetersäure allein oder mit Eisessig verdünnt hinzu.

Bei empfindlichen Substituenten kann die Nitrierung auch in einem Gemisch von Essigsäure oder Trifluoressigsäure mit Natriumnitrat durchgeführt werden. Hierzu suspendiert man das Pyridinderivat zusammen mit Natriumnitrat in Essig- bzw. Trifluoressigsäure und führt dann die Umsetzung durch.

Besonders bevorzugt ist die Nitrierung in Gegenwart von Essigsäureanhydrid. Hierbei gibt man die Salpetersäure zu dem in Essigsäureanhydrid gelösten oder suspendierten Pyridinderivat Ib und rührt bis zum Ende der Umsetzung nach. Zweckmäßig kann man beide Reaktionspartner - Pyridinderivat Ib und Salpetersäure - mit Eisessig verdünnen.

Bei Temperaturen oberhalb 20°C entsteht aus Mischungen von Salpetersäure und Essigsäureanhydrid Acetylnitrat.

Anstelle von Essigsäureanhydrid als Lösungsmittel kann auch Trifluoressigsäureanhydrid verwendet werden. Indifferente Lösungsmittel, die unter den jeweiligen Bedingungen der Nitrierung mit Salpetersäure nicht angegriffen werden, kann man ebenfalls verwenden. Bevorzugt werden aliphatische Chlorkohlenwasserstoffe wie Dichlormethan, Chloroform, Tetrachlormethan, 1,2-Dichlorethan und Polychlorethane. Geeignet sind aber auch Benzinfraktionen, Ligroin, Nitromethan, Acetonitril, Ethanol, Ether, Aceton, Mono- und Dichlorbenzole sowie Nitrobenzole.

Die Reaktion wird im allgemeinen bei einer Temperatur von -20 bis 400°C, vorzugsweise 0 bis 200°C, besonders bevorzugt 20 bis 120°C durchgeführt.

Die molaren Verhältnisse, in denen die benötigten Ausgangsverbindungen miteinander umgesetzt werden, betragen 0,9:1 bis 3,0:1, vorzugsweise 1,1:1 bis 1,5:1 für das Verhältnis von Salpetersäure zu Pyridinderivat Ib. Die Konzentration der Eddukte im Lösungsmittel beträgt 0,1 bis 5 mol/l, bevorzugt 0,3 bis 2 mol/l.

Im Falle exothermer Reaktionen hält man zweckmäßig bei der Zugabe der ersten Hälfte der Salpetersäure durch Kühlung eine Temperatur von 20 bis 40°C ein und kann dann bei zunehmend milderem Reaktionsverlauf das Kältebad entfernen. Bei nicht ausreichendem Umsetzungsgrad kann es erforderlich sein, durch Erwärmen, z.B. bis 120°C, die Reaktion zu Ende zu führen. Die Reaktionszeit beträgt - je nach vorgegebener Temperatur - eine viertel bis 24 Stunde (n).

Die Aufarbeitung kann man auf übliche Weise, z.B. durch Filtration, Waschen des Festgutes, Destillation von Filtrat und Waschfiltration, vornehmen. Vorteilhaft kann man die Pyridinderivate Ia in Wasser bzw. Eiswasser ausfällen, absaugen, mit wäßrigen alkalischen Lösungen von Säureresten befreien und wie beschrieben aufarbeiten.

Das Verfahren zur Herstellung der Verbindungen IIa besteht in der Umsetzung eines Pyridincarbonsäureanhydrids oder dessen N-Oxids IIb mit nitrierenden Agentien und wird auf gleiche Weise wie die oben beschriebene Umsetzung der Pyridinderivate Ib durchgeführt.

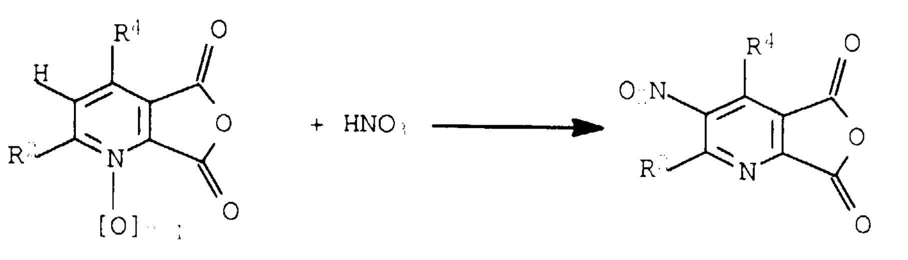

IIb IIa

Statt auf der Stufe der Pyridindicarbonsäureanhydride oder deren N-Oxide IIb kann die Nitrierung auch auf der entsprechenden Diester- oder deren N-Oxidstufe Vb durchgeführt werden. Diese können anschließend entweder durch Hydrolyse zu den Carbonsäuresalzen und Cyclisierung in die Anhydride IIa umgewandelt oder direkt mit dem Amin III zu Carbonesterhalbamiden VI umgesetzt werden, die nach Hydrolyse zu IV zu den erfindungsgemäßen Verbindungen Ia cyclisiert werden können.

Die Abspaltung des Pyridin-N-oxid-Sauerstoffs kann bereits unter den Bedingungen der Nitrierung in Essigsäureanhydrid als Reaktionsmedium erfolgen, anderenfalls kann er entsprechend den Bedingungen der Literatur (Houben-Weyl, Methoden der org. Chemie, G. Thieme Verlag, Stuttgart, Bd. 10/2 S. 714 (4. Auflage)) durch Einleiten von Stickstoffmonoxid oder durch einstündiges Erwärmen mit Phosphor-(III)-chlorid auf 70 bis 80°C entfernt werden. An gleicher Stelle entnimmt man auch eine Übersicht zur Darstellung von Pyridin-N-oxiden.

Das obengenannte Verfahren zur Herstellung von Pyridindicarbonsäureestern der Formel Vb, in der $R^2$ bis $R^4$ die vorgenannte Bedeutung besitzen, wobei $R^5$ einen beliebig substituierten Alkylrest darstellt,

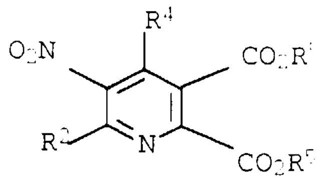

Vb

besteht in der Behandlung eines Pyridindicarbonsäureesters oder dessen N-Oxid der Formeln Vb', in der $R^2$, $R^4$ und $R^5$ die vorgenannte Bedeutung besitzen, mit nitrierenden Agentien und gegebenenfalls Entoxygenierung

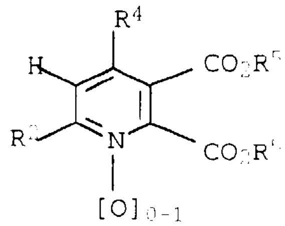

Vb'

Das Verfahren wird auf gleiche Weise wie die oben beschriebene Umsetzung der Pyridinderivate Ib durchgeführt. Daran schließen sich die oben aufgeführten Umwandlungen zu den Pyridinderivaten Ia an.

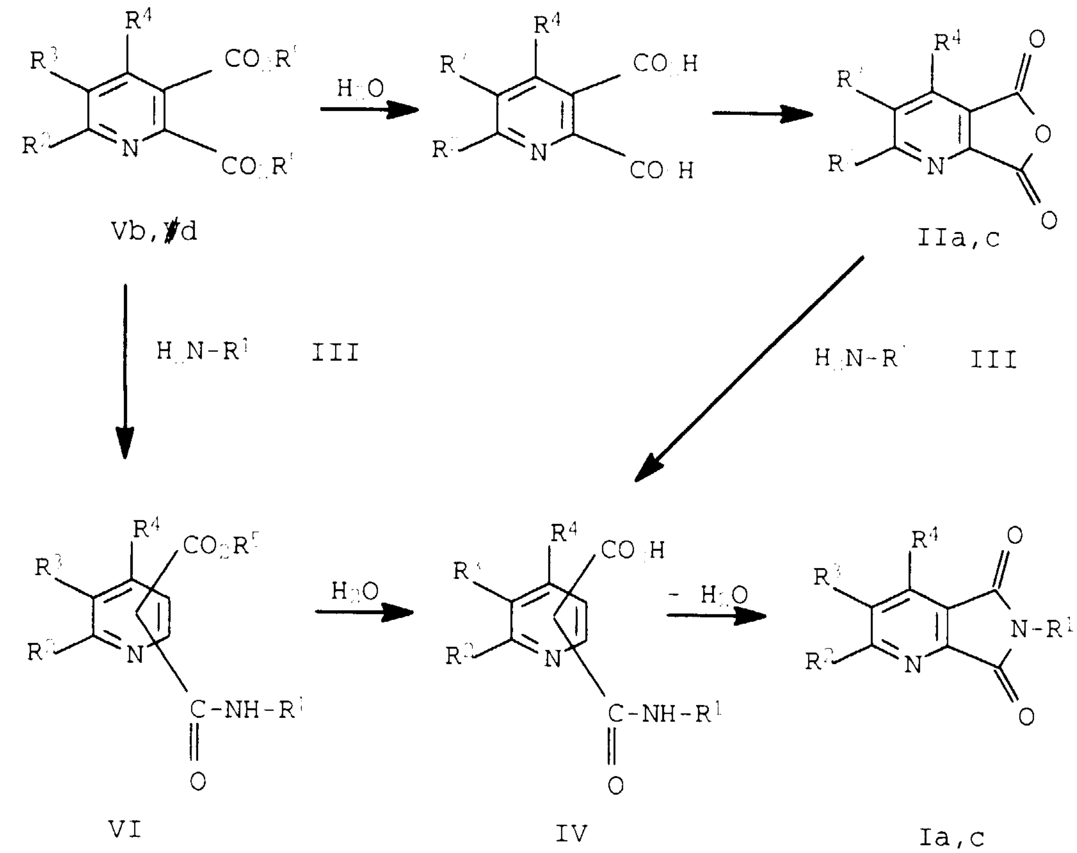


Vb,Vd IIa,c VI IV Ia,c

Vb) $R^3 = [NO_2]$ IIa: $R^3 = [NO_2]$

Vd) $R^3 - R^4 = [NH_2]_{1-3}$ IIc: $R^3 - R^4 = [NR^6R^7]_{1-3}$

Im Vergleich zu dem Stand der Technik sind die neuen nitrosubstituierten Pyridin-2,3-dicarbonsäureanhydride, -ester und -imide auf besonders einfache Weise in hohen Ausbeuten zugänglich. Nitrosubstituierte Pyridin-2,3-dicarbonsäureanhydride und -imide sind bisher nicht bekannt.

In der Reihe der Diester sind das 6-Methyl-5-nitro-, das 6-Chlor-5-nitro und das 6-Amino-5-nitro-derivat bekannt. Sie alle müssen in umständlicher Reaktion auf Basis von Ethoxymethylen-oxalessigester und einer Nitroketonkomponente aufgebaut werden. Im Falle des Nitroacetons liegt die Ausbeute bei nur 18 % (EP-A 227 932); darüber hinaus ist Nitroaceton nicht lagerfähig und kann sich spontan zersetzen (Beilstein HI, 661).

Im Falle der Nitrierung der 5-Chlor-6-hydroxynikotinsäure wird der Carbonsäurerest abgespalten und durch eine Nitrogruppe ersetzt (EP-A 467 308).

Vor dem Hintergrund der aus dem Stand der Technik vermittelten Lehre waren die erfindungsgemäßen Nitrierungen daher nicht zu erwarten. Überraschend ist auch, daß bei Einsatz der unsubstituierten N-Oxide nicht in 4-, sondern in der 5-Position nitriert wird. Nach Houben-Weyl, Methoden der org. Chemie, G. Thieme Verlag Stuttgart, Bd. 10/1 S. 713 (4. Auflage) hätte man hier eine Substitution in 4- oder 6-Stellung erwartet.

Im Rahmen der Herstellung der erfindungsgemäßen Verbindungen wurde weiterhin gefunden, daß man aminosubstituierte Pyridinderivate der Formel Ic, in der $R^2$ und $R^4$ die vorgenannte Bedeutung besitzen, erhält,

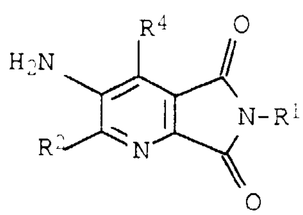

Ic

wenn man Pyridinderivate der Formel Ia einer Reduktion unterzieht.

Weiterhin wurde gefunden, daß man Pyridinderivate der Formel IIc

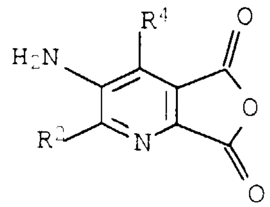

IIc

in der $R^2$ und $R^4$ die vorgenannte Bedeutung besitzen, wobei mindestens einer der Reste für Amino steht, erhält, wenn man Pyridinderivate der Formel IIa einer Reduktion unterzieht.

Statt auf der Stufe der Pyridindicarbonsäureanhydride IIa kann die Reduktion auch auf der entsprechenden Diesterstufe Vb durchgeführt werden. Diese können anschließend entweder durch Hydrolyse zu den Carbonsäuresalzen und Cyclisierung (gegebenenfalls unter Verwendung einer Aminschutzgruppe) in die Anhydride IIc umgewandelt oder direkt mit dem Amin III zu Carbonesterhalbamiden VI umgesetzt werden, die nach Hydrolyse zu IV zu den erfindungsgemäßen Verbindungen Ic (gegebenenfalls unter Verwendung einer Aminschutzgruppe) cyclisiert werden können.

Ein Verfahren zur Herstellung von Verbindungen Ic, besteht in der Reduktion eines Pyridinderivats Ia.

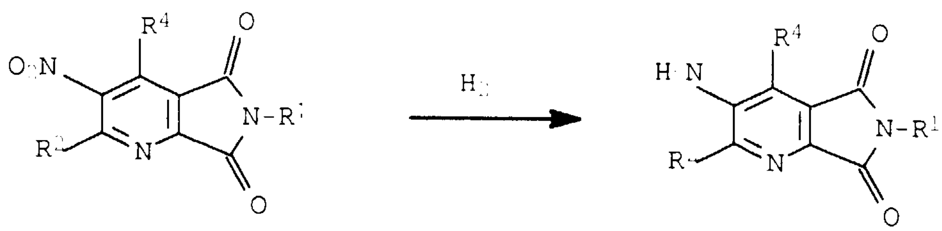

Ia Ic

Die Reduktion kann katalytisch mit Wasserstoff oder Hydrazinhydrat als Wasserstoffquelle an allen gängigen Katalysatoren drucklos oder unter Druck, kontinuierlich oder diskontinuierlich durchgeführt werden. Als Katalysatoren sind beispielsweise Pt, Pd, Re, Ni, Pd und Ru oder Gemische z.B. Ra/Ni, Pt/Ru-oxide geeignet. Weitere Katalysatoren sind in Houben-Weyl, Methoden der org. Chemie, G. Thieme Verlag, Stuttgart, Bd. 4/1c S. 506-537 (4. Auflage) angeführt, ebenso Promotoren und Acceleratoren zur Beschleunigung der Hydrierung. Reaktionsbedingungen, Wahl des Lösungsmittels, Transferbedingungen, Angaben zur selektiven Hydrierung in Gegenwart von beispielsweise Halogenen und Angaben zur Aufarbeitung findet man ebenda, ferner in Bd. 11/1 S. 360-381. Letzterer beschreibt auch die Reduktion mit Eisen (S. 394), mit Schwefelwasserstoff, Sulfiden und Polysulfiden (s. 409), ferner Zinn und Zinn-(II)-chlorid (S. 422), Natriumdithionit (S. 437), Eisen-(II)-hydroxyd (S. 443), Lithiumaluminiumhydrid (S. 447), Sulfiten (S. 457), Zink (S. 463), Aluminium (S. 469) oder eine elektrolytische Reduktion.

Ein Verfahren zur Herstellung von Pyridinderivaten der Formel IIc, besteht in der Reduktion eines Pyridinderivats IIa.

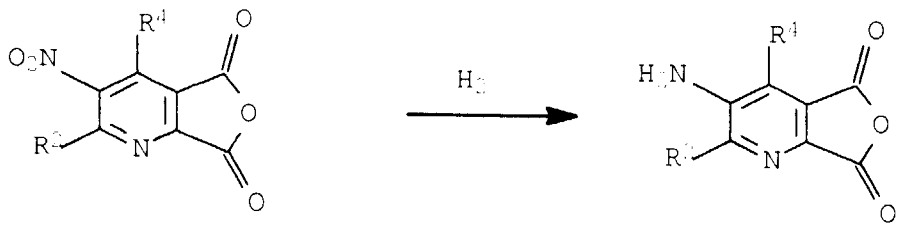


IIa IIc

Die Reduktion wird auf gleiche Weise wie die oben beschriebene Reduktion der Pyridinderivate Ia durchgeführt.

Ein Verfahren zur Herstellung von Pyridinderivaten der Formel Vd, besteht in der Reduktion eines Pyridinderivats Vb.

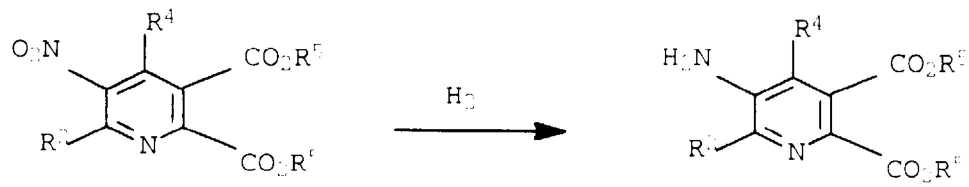


Vb Vd

Die Reduktion wird auf gleiche Weise wie die oben beschriebene Reduktion der Pyridinderivate Ia durchgeführt.

Ein weiteres Verfahren zur Herstellung von Pyridinderivaten der Formel Ve, worin mindestens einer der Reste $R^2$ bis $R^4$ für $NR^{6'}R^{7'}$ steht, wobei $R^{6'}$ und $R^{7'}$ unabhängig voneinander für Wasserstoff oder $C_1$-$C_4$-Alkyl, ferner $R^{6'}$ noch für Benzyl oder $C_1$-$C_4$-Alkoxy steht, besteht in der Umsetzung eines Pyridinderivats Vf mit einem Amin.

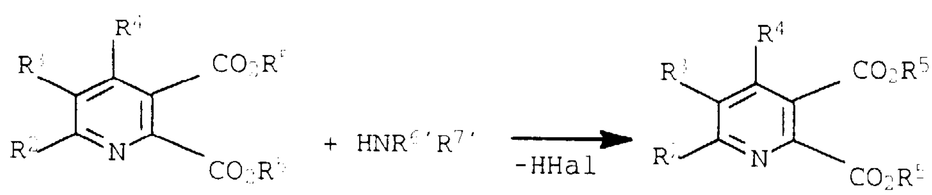


$[R^2–R^4 = (Hal)_{1-3}]$ $[R^2–R^4 = (NR^{6'}R^{7'})_{1-3}]$

Vf Ve

Hierzu legt man das Pyridinderivat Vf in einem der vorgenannten inerten Lösungsmittel vor und gibt dann das Amin, gasförmig oder flüssig, sowie eine Hilfsbase, hinzu.

Die Reaktion wird im allgemeinen bei einer Temperatur von -20 bis 250°C, vorzugsweise 0 bis 200°C, besonders bevorzugt 20 bis 180°C durchgeführt.

Die molaren Verhältnisse, in denen die benötigten Ausgangsverbindungen miteinander umgesetzt werden, betragen 0,9:1 bis 3,0:1, vorzugsweise 1,1:1 bis 1,5:1 für das Verhältnis von Amin zu Pyridinderivat Vf. Die Konzentration

der Eddukte im Lösungsmittel beträgt 0,1 bis 5 mol/l, bevorzugt 0,3 bis 2 mol/l.

Setzt man nur ungefähr stöchiometrische Mengen des Amins ein, so muß man zweckmäßig 0,9 bis 1,1 Äquivalente einer organischen Hilfsbase, bezogen auf den Ausgangsstoff Vf, zusetzen. Als Hilfsbase eignen sich organische Basen wie Trimethylamin, Triethylamin, N-Ethyl-diisopropylamin, Triisopropylamin, N,N-Dimethylanilin, N,N-Dimethylcyclohexylamin, N-Methylpyrrolidon, Pyridin, Chinolin, α,β,γ-Picolin, 2,4- und 2,6-Lutidin und Triethylendiamin.

Im Rahmen der Herstellung der erfindungsgemäßen Verbindungen wurde weiterhin gefunden, daß man aminosubstituierte Pyridinderivate der Formel Ic, in der $R^2$ bis $R^4$ die vorgenannte Bedeutung besitzen, wobei mindestens einer der Reste für Amino steht, erhält,

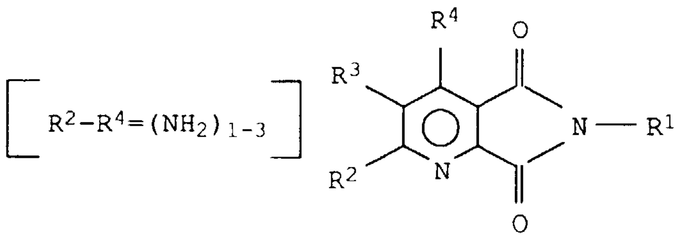

Ic

wenn man Pyridinderivate der Formel Id, in der $R^2$ bis $R^4$ die vorgenannte Bedeutung besitzen, wobei mindestens einer der Reste für Halogen steht, mit einem Metallcyanat umsetzt und das gebildete Isocyanat in situ hydrolisiert.

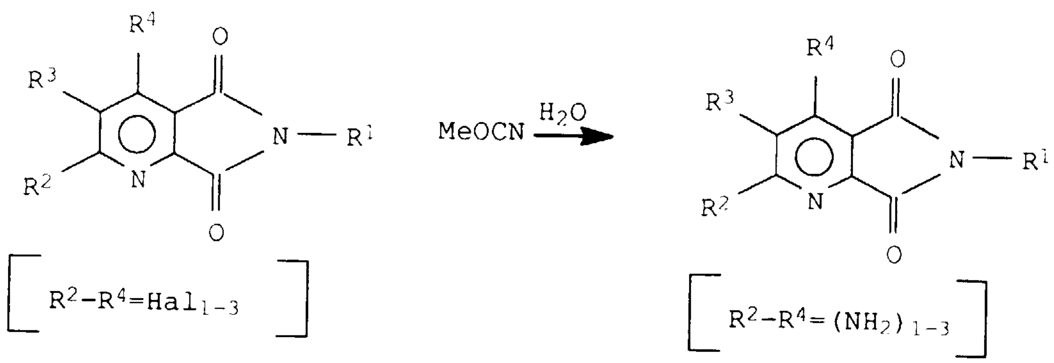


Id

Ic

Hierzu legt man das Pyridinderivat Id in einem der vorgenannten Lösungsmittel, zweckmäßig unter Zugabe von mindestens 1 bis 6 Äquivalenten, bevorzugt 2 bis 3 Äquivalenten Wasser vor, gibt das Metallcyanat hinzu und führt dann die Umsetzung durch.

Die Reaktion wird im allgemeinen bei einer Temperatur von 20 bis 200°C, bevorzugt 80 bis 150°C, besonders bevorzugt 100 bis 130°C durchgeführt.

Die molaren Verhältnisse, in denen das Pyridinderivat Id und das Metallcyanat miteinander umgesetzt werden, betragen 1:0,9 bis 1:3, vorzugsweise 1:1,5 bis 1:2,5. Die Konzentration der Edukte im Lösungsmittel beträgt 0,1 bis 5 mol/l, bevorzugt 0,3 bis 2 mol/l.

Als Cyanate sind die von Alkali-, Erdalkalimetallen sowie Eisen, Cobalt, Nickel, Kupfer und Zink abgeleiteten Salze geeignet, ferner das Ammoniumcyanat.

Zur Aufarbeitung extrahiert man das Umsetzungsgemisch mit Wasser zur Entfernung der Salze, trocknet und reinigt die organische Phase, z.B. durch Chromatographie oder Destillation. Die Umsetzungsprodukte sind jedoch meist genügend rein, so daß man nur von dem ausgefallenen Salz abzufiltrieren und die organische Phase einzuengen braucht.

Im Vergleich zu dem Stand der Technik sind die neuen aminosubstituierten Pyridin-2,3-dicarbonsäureanhydride, -ester und -imide auf besonders einfache Weise in hohen Ausbeuten zugänglich. Amino substituierte Pyridin-2,3-dicarbonsäureanhydride sind bisher nicht bekannt. In der Reihe der Imide wurde das 6-Amino-5-cyano-4-phenylpyridin-2,3-dicarbonsäureimid über eine Photoxygenierung und Ringschlußreaktion erschlossen (Synth. Commun. 22, 2053). Von den Estern wurde der 4-Amino-pyridin-2,3-dicarbonsäuredimethylester durch Veresterung der entsprechenden - aus einem Giftpilz isolierten - Säure bekannt [CA. 111, 112 292]. Der 5,6-Diaminopyridin-2,3-dicarbonsäurediethylester

wurde durch Reduktion eines analogen 5-Nitroesters hergestellt, wobei dieser mit einem Überschuß von 261 mol-% Palladium in Gegenwart von Wasserstoff durchgeführt wurde (EP-A 227 932). Ein 6-Amino-5-nitro-pyridin-2,3-dicarbonsäurediethylester wurde durch nucleophile Verdrängung eines entsprechenden 6-Chlorderivats mit Ammoniak gewonnen, wobei keine Angaben zur Ausbeute gemacht werden (EP-A 227 932). Der 5-Amino-6-methyl-pyridin-2,3-dicarbonsäuredimethyl- und diethylester wurden auf umständliche Weise durch Veresterung und Hofmann-Abbau eines schwierig zugänglichen Pyridintricarbonsäureamidderivats erhalten (Beilstein 4-22-006875).

Alle Verfahren sind aufwendige Mehrstufenreaktionen oder erfordern unwirtschaftliche Katalysatormengen.

Im Hinblick auf die bestimmungsgemäße Verwendung der Verbindungen I kommen als Substituenten beispielsweise folgende Reste in Betracht:

$R^1$

Wasserstoff;

$C_1$-$C_4$-Alkoxy wie Methoxy, Ethoxy, Propoxy, 1-Methylethoxy, Butoxy, 1-Methylpropoxy, 2-Methylpropoxy und 1,1-Dimethylethoxy, insbesondere Methoxy, Ethoxy, 1-Methylethoxy und 1,1-Dimethylethoxy;

$C_1$-$C_6$-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Propyl, 1-Methylethyl und 1,1-Dimethylethyl, wobei die genannten Reste eine bis drei der folgenden Gruppen tragen können:

$C_1$-$C_4$-Alkoxy wie oben genannt, insbesondere Methoxy und Ethoxy;

$C_1$-$C_4$-Halogenalkoxy wie Difluormethoxy, Trifluormethoxy, Chlordifluormethoxy, Dichlorfluormethoxy, 1-Fluorethory, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy und Pentafluorethoxy;

$C_1$-$C_4$-Alkylthio wie Methylthio, Ethylthio, Propylthio, 1-Methylethylthio, n-Butylthio, 1-Methylpropylthio, 2-Methylpropylthio und 1,1-Dimethylethylthio, insbesondere Methylthio und Ethylthio;

$C_1$-$C_4$-Halogenalkylthio wie Difluormethylthio, Trifluormethylthio, Chlordifluormethylthio, 1-Fluorethylthio, 2-Fluorethylthio, 2,2-Difluorethylthio, 2,2,2-Trifluorethylthio, 2-Chlor-2,2-difluorethylthio, 2,2-Dichlor-2-fluorethylthio, 2,2,2-Trichlorethylthio und Pentafluorethylthio, insbesondere Trifluormethylthio und Pentafluorethylthio;

($C_1$-$C_4$-Dialkyl)amino wie Dimethylamino, Diethylamino, Dipropylamino, Diisopropylamino, Dibutylamino, Methylethylamino, insbesondere Dimethylamino und Methylethylamino;

$C_3$-$C_8$-Cycloalkyl wie Cyclopropyl, Cyclobutyl, Cyclopentyl, Cyclohexyl, Cycloheptyl und Cyclooctyl, insbesondere Cyclopropyl, Cyclopentyl und Cyclohexyl;

Halogen wie Fluor, Chlor, Brom, Jod, insbesondere Fluor und Chlor;

$R^1$ ferner $C_3$-$C_8$-Cycloalkyl wie oben genannt, insbesondere Cyclopropyl, Cyclobutyl; Cyclopentyl und Cyclohexyl, das eine bis drei der folgenden Gruppen tragen kann: $C_1$-$C_6$-Alkyl wie oben genannt, insbesondere Methyl, Ethyl und Isopropyl; Halogenalkyl wie oben für die homologen Halogenalkylthioreste genannt, insbesondere Trifluormethyl; $C_1$-$C_4$-Alkoxy wie oben genannt, insbesondere Methoxy und Ethoxy; $C_1$-$C_4$-Halogenalkoxy wie oben genannt, insbesondere Trifluormethoxy; Halogen wie oben genannt, insbesondere Fluor und Chlor;

$C_3$-$C_6$-Alkenyl wie 2-Propenyl, 2-Methylethenyl, 2-Butenyl, 3-Butenyl, 1-Methyl-2-propenyl, 2-Methyl-2-propenyl, 2-Pentenyl, 3-Pentenyl, 4-Pentenyl, 3-Methyl-2-butenyl, 1-Methyl-2-butenyl, 2-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 1-, 2-, 3-, 4- oder 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl,
1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-pentenyl und Ethyl-2-methyl-2-pentenyl, insbesondere Ethenyl, 2-Propenyl, 1-Methyl-ethenyl, 2-Butenyl, 3-Butenyl, 1-Methylpropyl, 1-Methyl-2-propenyl, 2-Methyl-2-propenyl, welches ein- bis dreimal durch Halogen, insbesondere Fluor und Chlor substituiert sein kann;

$R^1$ ferner $C_3$-$C_6$-Alkinyl wie Propargyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 3-Methyl-1-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl 1-Methyl-3-pentinyl, 1-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, insbesondere 1-Methyl-2-propinyl und 1,1-Dimethyl-2-propinyl, welches ein- bis dreimal durch Halogen wie vorstehend genannt, insbesondere Fluor und Chlor und/oder einmal durch Phenyl substituiert sein kann;

$R^2$, $R^3$ bzw. $R^4$ mindestens einer der Reste $NR^6R^7$ steht und die übrigen Reste bedeuten:

Wasserstoff, Halogen wie unter $R^1$ genannt, insbesondere Fluor und Chlor; Cyano; $C_1$-$C_6$-Alkyl, wie unter $R^1$ genannt, insbesondere Methyl, Ethyl, Propyl, 1-Methylethyl und 1,1-Dimethylethyl, welches ein bis fünf Halogenatome, wie unter $R^1$ genannt, insbesondere Fluor und Chlor, und/oder einen oder zwei der folgenden Reste tragen kann: $C_1$-$C_4$-Alkoxy wie unter $R^1$ genannt, insbesondere Methoxy, Ethoxy, 1-Methylethoxy und 1,1-Dimethylethoxy; $C_1$-$C_4$-Halogenalkoxy wie unter $R^1$ genannt, insbesondere Halogenmethoxy wie Difluormethoxy und Trifluormethoxy; $C_1$-$C_4$-Alkylthio wie unter $R^1$ genannt, insbesondere Methylthio und Ethylthio; $C_1$-$C_4$-Halogenalkylthio, wie unter $R^1$ genannt, insbesondere Difluormethylthio und Trifluormethylthio;

$C_3$-$C_6$-Cycloalkyl wie unter $R^1$ genannt, insbesondere Cyclopropyl;

$R^2$, $R^3$ bzw. $R^4$ ferner Benzyl, das ein bis dreimal durch $C_1$-$C_4$-Alkyl wie unter $R^1$ genannt, insbesondere Methyl, Ethyl und 1-Methylethyl; $C_1$-$C_4$-Halogenalkyl wie unter $R^1$ genannt, insbesondere Trifluormethyl und Chlordifluormethyl; $C_1$-$C_4$-Alkoxy wie unter $R^1$ genannt, insbesondere Methoxy und Ethoxy; $C_1$-$C_4$-Halogenalkoxy wie unter $R^1$ genannt, insbesondere Trifluormethoxy, Trichlormethoxy und Pentafluorethoxy; $C_1$-$C_4$-Alkylthio wie unter $R^1$ genannt, insbesondere Methylthio und Ethylthio; $C_1$-$C_4$-Halgenalkylthio wie unter $R^1$ genannt, insbesondere Difluormethylthio, Trifluormethylthio und Pentafluormethylthio; Halogen insbesondere Fluor und Chlor; Cyano oder Nitro substituiert sein kann;

$R^2$, $R^3$ bzw. $R^4$ ferner $C_3$-$C_8$-Cycloalkyl, wie unter $R^1$ genannt, insbesondere Cyclopropyl, Cyclopentyl und Cyclohexyl; das ein- bis dreimal durch $C_1$-$C_4$-Alkyl wie unter $R^1$ genannt, insbesondere Methyl und Ethyl; oder Halogen wie unter $R^1$ genannt, insbesondere Fluor und Chlor, substituiert sein kann;

$C_2$-$C_6$-Alkenyl wie unter $R^1$ genannt, ferner 1-Ethenyl, 1-Propenyl, 1-Methyl-1-propenyl, 2-Methyl-1-propenyl, 1-Pentenyl, 1-Methyl-1-butenyl, 2-Methyl-1-butenyl, 1-Ethyl-1-propenyl, 1-Methyl-1-pentenyl, 2-Methyl-1-pentenyl, 3-Methyl-1-pentenyl, 4-Methyl-1-pentenyl, 1,2-Dimethyl-1-butenyl, 1,3-Dimethyl-1-butenyl, 2,3-Dimethyl-1-butenyl, 3,3-Dimethyl-1-butenyl, Ethyl-1-butenyl, 2-Ethyl-1-butenyl, 1-Ethyl-2-methyl-1-pentenyl, insbesondere Ethenyl, 1-Propenyl, 2-Propenyl, 1-Methylethenyl, 1-Butenyl, 2-Butenyl, 3-Butenyl, 1-Methylpropenyl, 1-Methyl-2-propenyl, 2-Methyl-1-propenyl, 2-Methyl-2-propenyl, welches ein bis dreimal durch Halogen wie unter $R^1$ genannt, insbesondere Fluor und Chlor; oder $C_1$-$C_3$-Alkoxy wie unter $R^1$ genannt, insbesondere Methoxy und Ethoxy; und/oder einmal durch Phenyl substituiert sein kann, wobei der Phenylrest seinerseits eine bis drei der folgenden Gruppen tragen kann: Alkyl wie unter $R^1$ genannt, insbesondere Methyl, Ethyl und 1-Methylethyl; Halogenalkyl wie unter $R^1$ genannt, insbesondere Trifluormethyl und Chlordifluormethyl; Alkoxy wie unter $R^1$ genannt, insbesondere Methoxy und Ethoxy; Halogenalkoxy wie unter $R^1$ genannt, insbesondere Trifluormethoxy, Trichlormethoxy und Pentafluorethoxy; Alkylthio wie unter $R^1$ genannt, insbesondere Methylthio und Ethylthio; Halogenalkylthio wie unter $R^1$ genannt, insbesondere Difluormethylthio, Trifluormethylthio und Pentafluormethylthio; Halogen wie unter $R^1$ genannt, insbesondere Fluor und Chlor; Cyano oder Nitro;

$C_2$-$C_6$-Alkinyl, wie unter $R^1$ genannt, ferner Ethinyl, 1-Propinyl, 1-Butinyl, 1-Pentinyl, 1-Hexinyl, 3-Methyl-1-pentinyl, 4-Methyl-1-pentinyl, insbesondere Ethinyl, 1-Propinyl und Propargyl, welches ein- bis dreimal durch Halogen wie vorstehend genannt, insbesondere Fluor und Chlor; oder Alkoxy wie vorstehend genannt, insbesondere Methoxy und Ethoxy; und/oder einmal durch Phenyl substituiert sein kann, wobei der Phenylrest seinerseits eine bis drei der folgenden Gruppen tragen kann: Alkyl wie vorstehend genannt, insbesondere Methyl, Ethyl und 1-Methylethyl; Halogenalkyl

wie vorstehend genannt, insbesondere Trifluormethyl und Chlordifluormethyl; Alkoxy wie vorstehend genannt, insbesondere Methoxy und Ethoxy; Halogenalkoxy wie vorstehend genannt, insbesondere Trifluormethoxy, Trichlormethoxy und Pentafluorethoxy; Alkylthio wie vorstehend genannt, insbesondere Methylthio und Ethylthio; Halogenalkylthio wie vorstehend genannt, insbesondere Difluormethylthio, Trifluormethylthio und Pentafluormethylthio; Halogen wie vorstehend genannt, insbesondere Fluor und Chlor, Cyano oder Nitro;

$R^2$, $R^3$ bzw. $R^4$ ferner $C_1$-$C_4$-Alkoxy oder -Alkylthio wie unter $R^1$ genannt, insbesondere Methoxy und Ethoxy, Methylthio und Ethylthio;

$C_1$-$C_4$-Halogenalkoxy oder -Halogenalkylthio wie vorstehend genannt, insbesondere Trifluormethoxy, Trichlormethoxy und Pentafluorethoxy, Difluormethylthio, Trifluormethylthio und Pentafluorethylthio;

$C_2$-$C_5$-Alkenyloxy wie Vinyloxy, 2-Propenyloxy, 1-Methyl-ethenyloxy, 2-Methyl-3-butenyloxy, insbesondere 2-Propenyloxy und 2-Methyl-3-butenyloxy;

$C_2$-$C_5$-Alkinyloxy wie Ethinyloxy, 2-Propinyloxy, 1-Methyl-ethinyloxy, 2-Methyl-3-butinyloxy, insbesondere 2-Propinyloxy und 2-Methyl-3-butinyloxy;

$C_1$-$C_4$-Alkylsulfinyl wie Methylsulfinyl, Ethylsulfinyl, n-Propylsulfinyl, i-Propylsulfinyl, n-Butylsulfinyl, tert.-Butylsulfinyl, insbesondere Methylsulfinyl;

$C_1$-$C_4$-Alkylsulfonyl wie Methylsulfonyl, Ethylsulfonyl, n-Propylsulfonyl, i-Propylsulfonyl, n-Butylsulfonyl, tert.-Butylsulfonyl, insbesondere Methylsulfonyl;

$C_1$-$C_4$-Halogenalkylsulfonyl wie Trifluormethylsulfonyl, Pentafluorethylsulfonyl, Monofluorbutylsulfonyl, insbesondere Trifluormethylsulfonyl;

Phenoxy oder Phenylthio, welches ein- bis dreimal durch $C_1$-$C_4$-Alkyl wie unter $R^1$ genannt, insbesondere Methyl,

Ethyl und iso-Propyl; Halogenalkyl wie unter $R^1$ genannt, insbesondere Trifluormethyl und Chlordifluormethyl; Alkoxy wie vorstehend genannt, insbesondere Methoxy und Ethoxy; Halogenalkoxy wie vorstehend genannt, insbesondere Trifluormethoxy, Trichlormethoxy und Pentafluorethoxy; Alkylthio wie vorstehend genannt, insbesondere Methylthio und Ethylthio; Halogenalkylthio wie vorstehend genannt, insbesondere Difluormethylthio, Trifluormethylthio und Pentafluormethylthio; Halogen wie vorstehend genannt, insbesondere Fluor und Chlor; Cyano oder Nitro; substituiert sein kann;

ein 5- bis 6-gliedriger gesättigter oder aromatischer heterocyclischer Rest, enthaltend ein oder zwei Heteroatome, ausgewählt aus der Gruppe Sauerstoff, Schwefel und Stickstoff wie Tetrahydrofuryl, Tetrahydropyranyl, Furyl, Thienyl, Thiazolyl, Pyrazolyl, Pyrrolyl, Pyridyl, Morpholino, Piperidino, Pyrimidyl, beispielsweise 2-Tetrahydrofuranyl, 3-Tetrahydrofuranyl, 2-Tetrahydrothienyl, 3-Tetrahydrothienyl, 2-Tetrahydropyranyl, 3-Tetrahydropyranyl, 4-Tetrahydropyranyl, 2-Furyl, 3-Furyl, 2-Thienyl, 3-Thienyl, 3-Isoxazolyl, 4-Isoxazolyl, 5-Isoxazolyl, 3-Isothiazolyl, 4-Isothiazolyl, 5-Isothiazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 5-Imidazolyl, 2-Pyrrolyl, 3-Pyrrolyl, 3-Pyrazolyl, 4-Pyrazolyl, 5-Pyrazolyl, 2-Pyridyl, 3-Pyridyl und 4-Pyridyl, der ein bis drei der folgenden Substituenten tragen kann: $C_1$-$C_3$-Alkyl wie vorstehend genannt, insbesondere Methyl und Ethyl, Halogen wie vorstehend genannt, insbesondere Fluor und Chlor, $C_1$-$C_3$-Alkoxy wie unter $R^1$ genannt, insbesondere Methoxy und Ethoxy; oder $C_1$-$C_4$-Alkoxycarbonyl wie Methoxycarbonyl und Ethoxycarbonyl, insbesondere Methoxycarbonyl;

Phenyl, welches eine bis drei der folgenden Gruppen tragen kann: $C_1$-$C_6$-Alkyl wie unter $R^1$ genannt, insbesondere Methyl, Ethyl und iso-Propyl; $C_1$-$C_6$-Halogenalkyl wie unter $R^1$ genannt, insbesondere Trifluormethyl und Chlordifluormethyl; $C_1$-$C_6$-Alkoxy wie unter $R^1$ genannt, insbesondere Methoxy und Ethoxy; $C_1$-$C_6$-Halogenalkoxy wie unter $R^1$ genannt, insbesondere Trifluormethoxy, Trichlormethoxy und Pentafluorethoxy; $C_1$-$C_6$-Alkylthio wie unter $R^1$ genannt, insbesondere Methylthio und Ethylthio; $C_1$-$C_6$-Halogenalkylthio wie unter $R^1$ genannt, insbesondere Difluormethylthio, Trifluormethylthio und Pentafluormethylthio; Halogen wie unter $R^1$ genannt, insbesondere Fluor und Chlor; Cyano oder Nitro;

$R^3$ außerdem $NO_2$;

$R^5$ Wasserstoff, $C_1$-$C_4$-Alkylcarbonyl, $C_1$-$C_4$-Halogenalkylcarbonyl, $C_1$-$C_4$-Alkoxycarbonyl, $C_1$-$C_4$-Alkoxy-$C_2$-$C_4$-alkoxycarbonyl, $C_1$-$C_4$-Alkylcarbamoyl, $C_1$-$C_4$-Dialkylcarbamoyl, $C_1$-$C_4$-Alkylsulfonyl, $C_1$-$C_4$-Halogenalkylsulfonyl, Sulfamoyl, $C_1$-$C_4$-Alkylaminosulfonyl, $C_1$-$C_4$-Dialkylaminosulfonyl, Phenylsulfonyl, welches ein- bis dreimal durch $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, Halogen, Cyano oder Nitro substituiert sein kann, wobei die in den Definitionen enthaltenen Alkyl-, Alkyloxy-, Halogenalkyl-, Alkylthio- und Alkylamino-Bestandteile in den einzelnen Resten den voranstehend angegebenen im einzelnen aufgelisteten Definitionen entsprechen;

$R^6$ Wasserstoff, $C_1$-$C_4$-Alkyl, insbesondere Methyl und Ethyl, Benzyl, $C_1$-$C_4$-Alkoxy, z.B. Methoxy und Ethoxy, oder zusammen mit $R^7$ C=S;

$R^7$ $C_1$-$C_4$-Alkyl, insbesondere Methyl und Ethyl, sowie die für $R^5$ aufgeführten Reste.

Beispiele für bevorzugte Restekombinationen im Pyridinring sind der nachstehenden Tabelle zu entnehmen, wobei $R^1$ einen Rest aus der Gruppe L1 bis L66 entspricht, insbesondere einem 1-(Cyclopropyl)-ethylrest oder einem Cyclopropylrest.

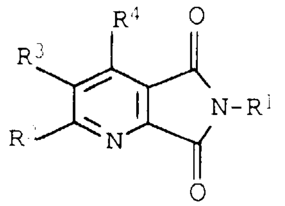

I

| $R^2$ | $R^3$ | $R^4$ |
|---|---|---|
| H | $NO_2$ | H |
| H | $NO_2$ | F |
| H | $NO_2$ | Cl |
| H | $NO_2$ | CN |
| H | $NO_2$ | $CH_3$ |

(fortgesetzt)

| $R^2$ | $R^3$ | $R^4$ |
|---|---|---|
| H | $NO_2$ | $CH_3O$ |
| H | $NO_2$ | $C_2H_5O$ |
| H | $NO_2$ | $CH_3S$ |
| H | $NO_2$ | $C_2H_5S$ |
| H | $NO_2$ | benzyl |
| H | $NO_2$ | $CF{=}CF_2$ |
| H | $NO_2$ | $C_2F_5$ |
| H | $NO_2$ | cyclopropyl |
| H | $NO_2$ | $C_2H_5$ |
| H | $NO_2$ | $CF_3O$ |
| H | $NO_2$ | $F_2CHO$ |
| H | $NO_2$ | $C_2F_5O$ |
| H | $NO_2$ | $CF_3S$ |
| H | $NO_2$ | $F_2CHS$ |
| H | $NO_2$ | $CF_3$ |

| $R^2$ | $R^3$ | $R^4$ |
|---|---|---|
| H | $NO_2$ | $CH_3SO$ |
| H | $NO_2$ | $CH_3SO_2$ |
| H | $NO_2$ | $CF_3SO_2$ |
| H | $NO_2$ | O-phenyl |
| H | $NO_2$ | O-(4-Cl-phenyl) |
| H | $NH_2$ | O-(3-$CF_3$-phenyl) |
| H | $NH_2$ | H |
| H | $NH_2$ | F |
| H | $NH_2$ | Cl |
| H | $NH_2$ | CN |
| H | $NH_2$ | $CH_3$ |
| H | $NH_2$ | $CH_3O$ |
| H | $NH_2$ | $C_2H_5O$ |
| H | $NH_2$ | $CH_3S$ |
| H | $NH_2$ | $C_2H_5S$ |
| H | $NH_2$ | benzyl |
| H | $NH_2$ | $CF{=}CF_2$ |
| H | $NH_2$ | $C_2F_5$ |
| H | $NH_2$ | cyclopropyl |
| H | $NH_2$ | $C_2H_5$ |

(fortgesetzt)

| $R^2$ | $R^3$ | $R^4$ |
|---|---|---|
| H | $NH_2$ | $CF_3O$ |
| H | $NH_2$ | $F_2CHO$ |
| H | $NH_2$ | $C_2F_5O$ |
| H | $NH_2$ | $CF_3S$ |
| H | $NH_2$ | $F_2CHS$ |
| H | $NH_2$ | $CF_3$ |
| H | $NH_2$ | $CH_3SO$ |
| H | $NH_2$ | $CH_3SO_2$ |
| H | $NH_2$ | $CF_3SO_2$ |
| H | $NH_2$ | O-phenyl |
| H | $NH_2$ | O-(4-Cl-phenyl) |
| H | $CH_3$(C=O)NH | O-(3-$CF_3$-Phenyl) |
| H | $CH_3$(C=O)NH | H |
| H | $CH_3$(C=O)NH | F |
| H | $CH_3$(C=O)NH | Cl |
| H | $CH_3$(C=O)NH | CN |
| H | $CH_3$(C=O)NH | $CH_3$ |
| H | $CH_3$(C=O)NH | $CH_3O$ |
| H | $CH_3$(C=O)NH | $C_2H_5O$ |

| $R^2$ | $R^3$ | $R^4$ |
|---|---|---|
| H | $CH_3$(C=O)NH | $CH_3S$ |
| H | $CH_3$(C=O)NH | $C_2H_5S$ |
| H | CH3(C=O)NH | benzyl |
| H | $CH_3$(C=O)NH | CF=$CF_2$ |
| H | $CH_3$(C=O)NH | $C_2F_5$ |
| H | $CH_3$(C=O)NH | cyclopropyl |
| H | $CH_3$(C=O)NH | $C_2H_5$ |
| H | $CH_3$(C=O)NH | $CF_3O$ |
| H | $CH_3$(C=O)NH | $F_2CHO$ |
| H | $CH_3$(C=O)NH | $C_2F_5O$ |
| H | $CH_3$(C=O)NH | $CF_3S$ |
| H | $CH_3$(C=O)NH | $F_2CHS$ |
| H | $CH_3$(C=O)NH | $CF_3$ |
| H | $CH_3$(C=O)NH | $CH_3SO$ |
| H | $CH_3$(C=O)NH | $CH_3SO_2$ |
| H | $CH_3$(C=O)NH | $CF_3SO_2$ |

(fortgesetzt)

| $R^2$ | $R^3$ | $R^4$ |
|---|---|---|
| H | $CH_3$(C=O)NH | O-phenyl |
| H | $CH_3$(C=O)NH | O-(4-Cl-phenyl) |
| H | $CF_3$(C=O)NH | O-(3-$CF_3$-Phenyl) |
| H | $CF_3$(C=O)NH | H |
| H | $CF_3$(C=O)NH | F |
| H | $CF_3$(C=O)NH | Cl |
| H | $CF_3$(C=O)NH | CN |
| H | $CF_3$(C=O)NH | $CH_3$ |
| H | $CF_3$(C=O)NH | $CH_3O$ |
| H | $CF_3$(C=O)NH | $C_2H_5O$ |
| H | $CF_3$(C=O)NH | $CH_3S$ |
| H | $CF_3$(C=O)NH | $C_2H_5S$ |
| H | $CF_3$(C=O)NH | benzyl |
| H | $CF_3$(C=O)NH | $CF=CF_2$ |
| H | $CF_3$(C=O)NH | $C_2F_5$ |
| H | $CF_3$(C=O)NH | cyclopropyl |
| H | $CF_3$(C=O)NH | $C_2H_5$ |
| H | $CF_3$(C=O)NH | $CF_3O$ |
| H | $CF_3$(C=O)NH | $F_2CHO$ |
| H | $CF_3$(C=O)NH | $C_2F_5O$ |
| H | $CF_3$(C=O)NH | $CF_3S$ |
| H | $CF_3$(C=O)NH | $F_2CHS$ |
| H | $CF_3$(C=O)NH | $CF_3$ |

| $R^2$ | $R^3$ | $R^4$ |
|---|---|---|
| H | $CF_3$(C=O)NH | $CH_3SO$ |
| H | $CF_3$(C=O)NH | $CH_3SO_2$ |
| H | $CF_3$(C=O)NH | $CF_3SO_2$ |
| H | $CF_3$(C=O)NH | O-phenyl |
| H | $CF_3$(C=O)NH | O-(4-Cl-phenyl) |
| H | $CH_3NH$ | O-(3-$CF_3$-phenyl) |
| H | $CH_3NH$ | H |
| H | $CH_3NH$ | F |
| H | $CH_3NH$ | Cl |
| H | $CH_3NH$ | CN |
| H | $CH_3NH$ | $CH_3$ |
| H | $CH_3NH$ | $CH_3O$ |

(fortgesetzt)

| $R^2$ | $R^3$ | $R^4$ |
|---|---|---|
| H | $CH_3NH$ | $C_2H_5O$ |
| H | $CH_3NH$ | $CH_3S$ |
| H | $CH_3NH$ | $C_2H_5S$ |
| H | $CH_3NH$ | benzyl |
| H | $CH_3NH$ | $CF{=}CF_2$ |
| H | $CH_3NH$ | $C_2F_5$ |
| H | $CH_3NH$ | cyclopropyl |
| H | $CH_3NH$ | $C_2H_5$ |
| H | $CH_3NH$ | $CF_3O$ |
| H | $CH_3NH$ | $F_2CHO$ |
| H | $CH_3NH$ | $C_2F_5O$ |
| H | $CH_3NH$ | $CF_3S$ |
| H | $CH_3NH$ | $F_2CHS$ |
| H | $CH_3NH$ | $CF_3$ |
| H | $CH_3NH$ | $CH_3SO$ |
| H | $CH_3NH$ | $CH_3SO_2$ |
| H | $CH_3NH$ | $CF_3SO_2$ |
| H | $CH_3NH$ | O-phenyl |
| H | $CH_3NH$ | O-(4-Cl-phenyl) |

| $R^2$ | $R^3$ | $R^4$ |
|---|---|---|
| F | $NO_2$ | $CH_3$ |
| F | $NO_2$ | $CF_3O$ |
| F | $NO_2$ | $F_2CHO$ |
| F | $NO_2$ | $C_2F_5O$ |
| F | $NO_2$ | $CF_3S$ |
| F | $NO_2$ | $F_2CHS$ |
| F | $NO_2$ | $CH_3SO$ |
| F | $NO_2$ | $CH_3SO_2$ |

| $R^2$ | $R^3$ | $R^4$ |
|---|---|---|
| F | $NO_2$ | $CF_3SO_2$ |
| F | $NO_2$ | O-phenyl |
| F | $NO_2$ | O-(4-Cl-phenyl) |
| F | $NO_2$ | $ClCF_2O$ |
| F | $NO_2$ | i-$C_3H_7$ |
| F | $NO_2$ | i-$C_4H_9$ |

(fortgesetzt)

| $R^2$ | $R^3$ | $R^4$ |
|---|---|---|
| Cl | $NO_2$ | O-(3-$CF_3$-phenyl) |
| Cl | $NO_2$ | H |
| Cl | $NO_2$ | F |
| Cl | $NO_2$ | Cl |
| Cl | $NO_2$ | CN |
| Cl | $NO_2$ | $CH_3$ |
| Cl | $NO_2$ | $CH_3O$ |
| Cl | $NO_2$ | $C_2H_5O$ |
| Cl | $NO_2$ | $CH_3S$ |
| Cl | $NO_2$ | $C_2H_5S$ |
| Cl | $NO_2$ | benzyl |
| Cl | $NO_2$ | $CF{=}CF_2$ |
| Cl | $NO_2$ | $C_2F_5$ |
| Cl | $NO_2$ | cyclopropyl |
| Cl | $NO_2$ | $C_2H_5$ |
| Cl | $NO_2$ | $CF_3O$ |
| Cl | $NO_2$ | $F_2CHO$ |
| Cl | $NO_2$ | $C_2F_5O$ |
| Cl | $NO_2$ | $CF_3S$ |
| Cl | $NO_2$ | $F_2CHS$ |
| Cl | $NO_2$ | $CF_3$ |
| Cl | $NO_2$ | $CH_3SO$ |
| Cl | $NO_2$ | $CH_3SO_2$ |
| Cl | $NO_2$ | $CF_3SO_2$ |
| Cl | $NO_2$ | O-phenyl |
| Cl | $NO_2$ | O-(4-Cl-phenyl) |
| $CH_3O$ | $N_O2$ | O-(3-$CF_3$-phenyl) |
| $CH_3O$ | $NO_2$ | H |
| $CH_3O$ | $NO_2$ | F |
| $CH_3O$ | $NO_2$ | Cl |
| $CH_3O$ | $NO_2$ | CN |
| $CH_3O$ | $NO_2$ | $CH_3$ |
| $CH_3O$ | $NO_2$ | $CH_3O$ |
| $CH_3O$ | $NO_2$ | $C_2H_5O$ |
| $CH_3O$ | $NO_2$ | $CH_3S$ |

(continued)

| $R^2$ | $R^3$ | $R^4$ |
|---|---|---|
| $CH_3O$ | $NO_2$ | $C_2H_5S$ |
| $CH_3O$ | $NO_2$ | benzyl |
| $CH_3O$ | $NO_2$ | $CF=CF_2$ |
| $CH_3O$ | $NO_2$ | $C_2F_5$ |
| $CH_3O$ | $NO_2$ | cyclopropyl |
| $CH_3O$ | $NO_2$ | $C_2H_5$ |
| $CH_3O$ | $NO_2$ | $CF_3O$ |
| $CH_3O$ | $NO_2$ | $F_2CHO$ |
| $CH_3O$ | $NO_2$ | $C_2F_5O$ |
| $CH_3O$ | $NO_2$ | $CF_3S$ |
| $CH_3O$ | $NO_2$ | $F_2CHS$ |
| $CH_3O$ | $NO_2$ | $CF_3$ |
| $CH_3O$ | $NO_2$ | $CH_3SO$ |
| $CH_3O$ | $NO_2$ | $CH_3SO_2$ |
| $CH_3O$ | $NO_2$ | $CF_3SO_2$ |
| $CH_3O$ | $NO_2$ | O-phenyl |
| $CH_3O$ | $NO_2$ | O-(4-Cl-phenyl) |
| $CH_3$ | $NO_2$ | O-(3-$CF_3$-phenyl) |
| $CH_3$ | $NO_2$ | H |
| $CH_3$ | $NO_2$ | F |
| $CH_3$ | $NO_2$ | Cl |
| $CH_3$ | $NO_2$ | CN |
| $CH_3$ | $NO_2$ | $CH_3$ |
| $CH_3$ | $NO_2$ | $CH_3O$ |
| $CH_3$ | $NO_2$ | $C_2H_5O$ |
| $CH_3$ | $NO_2$ | $CH_3S$ |
| $CH_3$ | $NO_2$ | $C_2H_5S$ |
| $CH_3$ | $NO_2$ | benzyl |
| $CH_3$ | $NO_2$ | $CF=CF_2$ |
| $CH_3$ | $NO_2$ | $C_2F_5$ |
| $CH_3$ | $NO_2$ | cyclopropyl |
| $CH_3$ | $NO_2$ | $C_2H_5$ |
| $CH_3$ | $NO_2$ | $CF_3O$ |
| $CH_3$ | $NO_2$ | $F_2CHO$ |
| $CH_3$ | $NO_2$ | $C_2F_5O$ |
| $CH_3$ | $NO_2$ | $CF_3S$ |
| $CH_3$ | $NO_2$ | $F_2CHS$ |

| $R^2$ | $R^3$ | $R^4$ |
|---|---|---|
| $CH_3$ | $NO_2$ | $CF_3$ |
| $CH_3$ | $NO_2$ | $CH_3SO$ |
| $CH_3$ | $NO_2$ | $CH_3SO_2$ |
| $CH_3$ | $NO_2$ | $CF_3SO_2$ |
| $CH_3$ | $NO_2$ | O-phenyl |
| $CH_3$ | $NO_2$ | O-(4-Cl-phenyl) |
| F | $NH_2$ | $C_2H_5$ |
| F | $NH_2$ | $CF_3O$ |
| F | $NH_2$ | $F_2CHO$ |
| F | $NH_2$ | $C_2H_5O$ |
| F | $NH_2$ | $CF_3S$ |
| F | $NH_2$ | $CH_3SO$ |
| F | $NH_2$ | $CH_3SO_2$ |
| F | $NH_2$ | $CF_3SO_2$ |
| F | $NH_2$ | O-phenyl |
| F | $NH_2$ | O-(4-Cl-phenyl) |
| F | $NH_2$ | $ClCF_2O$ |
| F | $NH_2$ | i-$C_3H_7$ |
| F | $NH_2$ | CN |
| Cl | $CF_3$(C=O)NH | O-(3-$CF_3$-phenyl) |
| Cl | $CF_3$(C=O)NH | H |
| Cl | $CF_3$(C=O)NH | F |
| Cl | $CF_3$(C=O)NH | Cl |
| Cl | $CF_3$(C=O)NH | CN |
| Cl | $CF_3$(C=O)NH | $CH_3$ |
| Cl | $CF_3$(C=O)NH | $CH_3O$ |
| Cl | $CF_3$(C=O)NH | $C_2H_5O$ |
| Cl | $CF_3$(C=O)NH | $CH_3S$ |
| Cl | $CF_3$(C=O)NH | $C_2H_5S$ |
| Cl | $CF_3$(C=O)NH | benzyl |
| Cl | $CF_3$(C=O)NH | $CF=CF_2$ |
| Cl | $CF_3$(C=O)NH | $C_2F_5$ |
| Cl | $CF_3$(C=O)NH | cyclopropyl |
| Cl | $CF_3$(C=O)NH | $C_2H_5$ |
| Cl | $CF_3$(C=O)NH | $CF_3O$ |
| Cl | $CF_3$(C=O)NH | $F_2CHO$ |
| Cl | $CF_3$(C=O)NH | $C_2F_5O$ |
| Cl | $CF_3$(C=O)NH | $CF_3S$ |

(fortgesetzt)

| $R^2$ | $R^3$ | $R^4$ |
|---|---|---|
| Cl | $CF_3(C{=}O)NH$ | $F_2CHS$ |

| $R^2$ | $R^3$ | $R^4$ |
|---|---|---|
| Cl | $CF_3(C{=}O)NH$ | $CF_3$ |
| Cl | $CF_3(C{=}O)NH$ | $CH_3SO$ |
| Cl | $CF_3(C{=}O)NH$ | $CH_3SO_2$ |
| Cl | $CF_3(C{=}O)NH$ | $CF_3SO_2$ |
| Cl | $CF_3(C{=}O)NH$ | O-phenyl |
| Cl | $CF_3(C{=}O)NH$ | O-(4-Cl-phenyl) |
| Cl | $CH_3NH$ | O-(3-$CF_3$-phenyl) |
| Cl | $CH_3NH$ | H |
| Cl | $CH_3NH$ | F |
| Cl | $CH_3NH$ | Cl |
| Cl | $CH_3NH$ | CN |
| Cl | $CH_3NH$ | $CH_3$ |
| Cl | $CH_3NH$ | $CH_3O$ |
| Cl | $CH_3NH$ | $C_2H_5O$ |
| Cl | $CH_3NH$ | $CH_3S$ |
| Cl | $CH_3NH$ | $C_2H_5S$ |
| Cl | $CH_3NH$ | benzyl |
| Cl | $CH_3NH$ | $CF{=}CF_2$ |
| Cl | $CH_3NH$ | $C_2F_5$ |
| Cl | $CH_3NH$ | cyclopropyl |
| Cl | $CH_3NH$ | $C_2H_5$ |
| Cl | $CH_3NH$ | $CF_3O$ |
| Cl | $CH_3NH$ | $F_2CHO$ |
| Cl | $CH_3NH$ | $C_2F_5O$ |
| Cl | $CH_3NH$ | $CF_3S$ |
| Cl | $CH_3NH$ | $F_2CHS$ |
| Cl | $CH_3NH$ | $CF_3$ |
| Cl | $CH_3NH$ | $CH_3SO$ |
| Cl | $CH_3NH$ | $CH_3SO_2$ |
| Cl | $CH_3NH$ | $CF_3SO_2$ |
| Cl | $CH_3NH$ | O-phenyl |
| Cl | $CH_3NH$ | O-(4-Cl-phenyl) |
| $CH_3$ | $CF_3(C{=}O)NH$ | O-(3-$CF_3$-phenyl) |
| $CH_3$ | $CF_3(C{=}O)NH$ | H |

(fortgesetzt)

| $R^2$ | $R^3$ | $R^4$ |
|---|---|---|
| $CH_3$ | $CF_3(C=O)NH$ | F |
| $CH_3$ | $CF_3(C=O)NH$ | Cl |
| $CH_3$ | $CF_3(C=O)NH$ | CN |
| $CH_3$ | $CF_3(C=O)NH$ | $CH_3$ |
| $CH_3$ | $CF_3(C=O)NH$ | $CH_3O$ |

| $R^2$ | $R^3$ | $R^4$ |
|---|---|---|
| $CH_3$ | $CF_3(C=O)NH$ | $C_2H_5O$ |
| $CH_3$ | $CF_3(C=O)NH$ | $CH_3S$ |
| $CH_3$ | $CF_3(C=O)NH$ | $C_2H_5S$ |
| $CH_3$ | $CF_3(C=O)NH$ | benzyl |
| $CH_3$ | $CF_3(C=O)NH$ | $CF=CF_2$ |
| $CH_3$ | $CF_3(C=O)NH$ | $C_2F_5$ |
| $CH_3$ | $CF_3(C=O)NH$ | cyclopropyl |
| $CH_3$ | $CF_3(C=O)NH$ | $C_2H_5$ |
| $CH_3$ | $CF_3(C=O)NH$ | $CF_3O$ |
| $CH_3$ | $CF_3(C=O)NH$ | $F_2CHO$ |
| $CH_3$ | $CF_3(C=O)NH$ | $C_2F_5O$ |
| $CH_3$ | $CF_3(C=O)NH$ | $CF_3S$ |
| $CH_3$ | $CF_3(C=O)NH$ | $F_2CHS$ |
| $CH_3$ | $CF_3(C=O)NH$ | $CF_3$ |
| $CH_3$ | $CF_3(C=O)NH$ | $CH_3SO$ |
| $CH_3$ | $CF_3(C=O)NH$ | $CH_3SO_2$ |
| $CH_3$ | $CF_3(C=O)NH$ | $CF_3SO_2$ |
| $CH_3$ | $CF_3(C=O)NH$ | O-phenyl |
| $CH_3$ | $CF_3(C=O)NH$ | O-(4-Cl-phenyl) |
| $CH_3O$ | $NH_2$ | O-(3-$CF_3$-Phenyl) |
| $CH_3O$ | $NH_2$ | H |
| $CH_3O$ | $NH_2$ | F |
| $CH_3O$ | $NH_2$ | Cl |
| $CH_3O$ | $NH_2$ | CN |
| $CH_3O$ | $NH_2$ | $CH_3$ |
| $CH_3O$ | $NH_2$ | $CH_3O$ |
| $CH_3O$ | $NH_2$ | $C_2H_5O$ |
| $CH_3O$ | $NH_2$ | $CH_3S$ |
| $CH_3O$ | $NH_2$ | $C_2H_5S$ |
| $CH_3O$ | $NH_2$ | benzyl |

(fortgesetzt)

| $R^2$ | $R^3$ | $R^4$ |
|---|---|---|
| $CH_3O$ | $NH_2$ | $CF{=}CF_2$ |
| $CH_3O$ | $NH_2$ | $C_2F_5$ |
| $CH_3O$ | $NH_2$ | cyclopropyl |
| $CH_3O$ | $NH_2$ | $C_2H_5$ |
| $CH_3O$ | $NH_2$ | $CF_3O$ |
| $CH_3O$ | $NH_2$ | $F_2CHO$ |
| $CH_3O$ | $NH_2$ | $C_2F_5O$ |
| $CH_3O$ | $NH_2$ | $CF_3S$ |
| $CH_3O$ | $NH_2$ | $F_2CHS$ |

| $R^2$ | $R^3$ | $R^4$ |
|---|---|---|
| $CH_3O$ | $NH_2$ | $CF_3$ |
| $CH_3O$ | $NH_2$ | $CH_3SO$ |
| $CH_3O$ | $NH_2$ | $CH_3SO_2$ |
| $CH_3O$ | $NH_2$ | $CF_3SO_2$ |
| $CH_3O$ | $NH_2$ | O-phenyl |
| $CH_3O$ | $NH_2$ | O-(4-Cl-phenyl) |
| $CH_3O$ | $CF_3(C{=}O)NH$ | O-(3-$CF_3$-Phenyl) |
| $CH_3O$ | $CF_3(C{=}O)NH$ | H |
| $CH_3O$ | $CF_3(C{=}O)NH$ | F |
| $CH_3O$ | $CF_3(C{=}O)NH$ | Cl |
| $CH_3O$ | $CF_3(C{=}O)NH$ | CN |
| $CH_3O$ | $CF_3(C{=}O)NH$ | $CH_3$ |
| $CH_3O$ | $CF_3(C{=}O)NH$ | $CH_3O$ |
| $CH_3O$ | $CF_3(C{=}O)NH$ | $C_2H_5O$ |
| $CH_3O$ | $CF_3(C{=}O)NH$ | $CH_3S$ |
| $CH_3O$ | $CF_3(C{=}O)NH$ | $C_2H_5S$ |
| $CH_3O$ | $CF_3(C{=}O)NH$ | benzyl |
| $CH_3O$ | $CF_3(C{=}O)NH$ | CF=CF2 |
| $CH_3O$ | $CF_3(C{=}O)NH$ | $C_2F_5$ |
| $CH_3O$ | $CF_3(C{=}O)NH$ | cyclopropyl |
| $CH_3O$ | $CF_3(C{=}O)NH$ | $C_2H_5$ |
| $CH_3O$ | $CF_3(C{=}O)NH$ | $CF_3O$ |
| $CH_3O$ | $CF_3(C{=}O)NH$ | $F_2CHO$ |
| $CH_3O$ | $CF_3(C{=}O)NH$ | $C_2F_5O$ |
| $CH_3O$ | $CF_3(C{=}O)NH$ | $CF_3S$ |
| $CH_3O$ | $CF_3(C{=}O)NH$ | $F_2CHS$ |

(fortgesetzt)

| $R^2$ | $R^3$ | $R^4$ |
|---|---|---|
| $CH_3O$ | $CF_3$(C=O)NH | $CF_3$ |
| $CH_3O$ | $CF_3$(C=O)NH | $CH_3SO$ |
| $CH_3O$ | $CF_3$(C=O) NH | $CH_3SO_2$ |
| $CH_3O$ | $CF_3$(C=O)NH | $CF_3SO_2$ |
| $CH_3O$ | $CF_3$(C=O)NH | O-phenyl |
| $CH_3O$ | $CF_3$(C=O)NH | O-(4-Cl-phenyl) |
| $CH_3$ | $NH_2$ | O-(3-$CF_3$-phenyl) |
| $CH_3$ | $NH_2$ | H |
| $CH_3$ | $NH_2$ | F |
| $CH_3$ | $NH_2$ | Cl |
| $CH_3$ | $NH_2$ | CN |
| $CH_3$ | $NH_2$ | $CH_3$ |
| $CH_3$ | $NH_2$ | $CH_3O$ |

| $R^2$ | $R^3$ | $R^4$ |
|---|---|---|
| $CH_3$ | $NH_2$ | $C_2H_5O$ |
| $CH_3$ | $NH_2$ | $CH_3S$ |
| $CH_3$ | $NH_2$ | $C_2H_5S$ |
| $CH_3$ | $NH_2$ | benzyl |
| $CH_3$ | $NH_2$ | $CF=CF_2$ |
| $CH_3$ | $NH_2$ | $C_2F_5$ |
| $CH_3$ | $NH_2$ | cyclopropyl |
| $CH_3$ | $NH_2$ | $C_2H_5$ |
| $CH_3$ | $NH_2$ | $CF_3O$ |
| $CH_3$ | $NH_2$ | $F_2CHO$ |
| $CH_3$ | $NH_2$ | $C_2F_5O$ |
| $CH_3$ | $NH_2$ | $CF_3S$ |
| $CH_3$ | $NH_2$ | $F_2CHS$ |
| $CH_3$ | $NH_2$ | $CF_3$ |
| $CH_3$ | $NH_2$ | $CH_3SO$ |
| $CH_3$ | $NH_2$ | $CH_3SO_2$ |
| $CH_3$ | $NH_2$ | $CF_3SO_2$ |
| $CH_3$ | $NH_2$ | O-phenyl |
| $CH_3$ | $NH_2$ | O-(4-Cl-phenyl) |
| Cl | $NH_2$ | O-(3-$CF_3$-phenyl) |
| Cl | $NH_2$ | H |
| Cl | $NH_2$ | F |

(fortgesetzt)

| $R^2$ | $R^3$ | $R^4$ |
|---|---|---|
| Cl | $NH_2$ | Cl |
| Cl | $NH_2$ | CN |
| Cl | $NH_2$ | $CH_3$ |
| Cl | $NH_2$ | $CH_3O$ |
| Cl | $NH_2$ | $C_2H_5O$ |
| Cl | $NH_2$ | $CH_3S$ |
| Cl | $NH_2$ | $C_2H_5S$ |
| Cl | $NH_2$ | benzyl |
| Cl | $NH_2$ | $CF{=}CF_2$ |
| Cl | $NH_2$ | $C_2F_5$ |
| Cl | $NH_2$ | cyclopropyl |
| Cl | $NH_2$ | $C_2H_5$ |
| Cl | $NH_2$ | $CF_3O$ |
| Cl | $NH_2$ | $F_2CHO$ |
| Cl | $NH_2$ | $C_2F_5O$ |
| Cl | $NH_2$ | $CF_3S$ |
| Cl | $NH_2$ | $F_2CHS$ |

| $R^2$ | $R^3$ | $R^4$ |
|---|---|---|
| Cl | $NH_2$ | $CF_3$ |
| Cl | $NH_2$ | $CH_3SO$ |
| Cl | $NH_2$ | $CH_3SO_2$ |
| Cl | $NH_2$ | $CF_3SO_2$ |
| Cl | $NH_2$ | O-phenyl |
| Cl | $NH_2$ | O-(4-Cl-phenyl) |
| $NH_2$ | $NO_2$ | O-(3-$CF_3$-phenyl ) |
| $NH_2$ | $NO_2$ | H |
| $NH_2$ | $NO_2$ | F |
| $NH_2$ | $NO_2$ | Cl |
| $NH_2$ | $NO_2$ | CN |
| $NH_2$ | $NO_2$ | $CH_3$ |
| $NH_2$ | $NO_2$ | $CH_3O$ |
| $NH_2$ | $NO_2$ | $C_2H_5O$ |
| $NH_2$ | $NO_2$ | $CH_3S$ |
| $NH_2$ | $NO_2$ | $C_2H_5S$ |
| $NH_2$ | $NO_2$ | benzyl |
| $NH_2$ | $NO_2$ | $CF{=}CF_2$ |

(fortgesetzt)

| $R^2$ | $R^3$ | $R^4$ |
|---|---|---|
| $NH_2$ | $NO_2$ | $C_2F_5$ |
| $NH_2$ | $NO_2$ | cyclopropyl |
| $NH_2$ | $NO_2$ | $C_2H_5$ |
| $NH_2$ | $NO_2$ | $CF_3O$ |
| $NH_2$ | $NO_2$ | $F_2CHO$ |
| $NH_2$ | $NO_2$ | $C_2F_5O$ |
| $NH_2$ | $NO_2$ | $CF_3S$ |
| $NH_2$ | $NO_2$ | $F_2CHS$ |
| $NH_2$ | $NO_2$ | $CF_3$ |
| $NH_2$ | $NO_2$ | $CH_3SO$ |
| $NH_2$ | $NO_2$ | $CH_3SO_2$ |
| $NH_2$ | $NO_2$ | $CF_3SO_2$ |
| $NH_2$ | $NO_2$ | O-phenyl |
| $NH_2$ | $NO_2$ | O-(4-Cl-phenyl) |
| $NH_2$ | Cl | O-(3-$CF_3$-phenyl) |
| $NH_2$ | Cl | H |
| $NH_2$ | Cl | F |
| $NH_2$ | Cl | Cl |
| $NH_2$ | Cl | CN |
| $NH_2$ | Cl | $CH_3$ |
| $NH_2$ | Cl | $CH_3H$ |

| $R^2$ | $R^3$ | $R^4$ |
|---|---|---|
| $NH_2$ | Cl | $C_2H_5O$ |
| $NH_2$ | Cl | $CH_3S$ |
| $NH_2$ | Cl | $C_2H_5S$ |
| $NH_2$ | Cl | benzyl |
| $NH_2$ | Cl | $CF=CF_2$ |
| $NH_2$ | Cl | $C_2F_5$ |
| $NH_2$ | Cl | cyclopropyl |
| $NH_2$ | Cl | $C_2H_5$ |
| $NH_2$ | Cl | $CF_3O$ |
| $NH_2$ | Cl | $F_2CHO$ |
| $NH_2$ | Cl | $C_2F_5O$ |
| $NH_2$ | Cl | $CF_3S$ |
| $NH_2$ | Cl | $F_2CHS$ |
| $NH_2$ | Cl | $CF_3$ |

(fortgesetzt)

| $R^2$ | $R^3$ | $R^4$ |
|---|---|---|
| $NH_2$ | Cl | $CH_3SO$ |
| $NH_2$ | Cl | $CH_3SO_2$ |
| $NH_2$ | Cl | $CF_3SO_2$ |
| $NH_2$ | Cl | O-phenyl |
| $NH_2$ | Cl | O-(4-Cl-phenyl) |
| $NH_2$ | $NH_2$ | O-(3-$CF_3$-Phenyl) |
| $NH_2$ | $NH_2$ | H |
| $NH_2$ | $NH_2$ | F |
| $NH_2$ | $NH_2$ | Cl |
| $NH_2$ | $NH_2$ | CN |
| $NH_2$ | $NH_2$ | $CH_3$ |
| $NH_2$ | $NH_2$ | $CH_3O$ |
| $NH_2$ | $NH_2$ | $C_2H_5O$ |
| $NH_2$ | $NH_2$ | $CH_3S$ |
| $NH_2$ | $NH_2$ | $C_2H_5S$ |
| $NH_2$ | $NH_2$ | benzyl |
| $NH_2$ | $NH_2$ | $CF{=}CF_2$ |
| $NH_2$ | $NH_2$ | $C_2F_5$ |
| $NH_2$ | $NH_2$ | cyclopropyl |
| $NH_2$ | $NH_2$ | $C_2H_5$ |
| $NH_2$ | $NH_2$ | $CF_3O$ |
| $NH_2$ | $NH_2$ | $F_2CHO$ |
| $NH_2$ | $NH_2$ | $C_2F_5O$ |
| $NH_2$ | $NH_2$ | $CF_3S$ |
| $NH_2$ | $NH_2$ | $F_2CHS$ |

| $R^2$ | $R^3$ | $R^4$ |
|---|---|---|
| $NH_2$ | $NH_2$ | $CF_3$ |
| $NH_2$ | $NH_2$ | $CH_3SO$ |
| $NH_2$ | $NH_2$ | $CH_3SO_2$ |
| $NH_2$ | $NH_2$ | $CF_3SO_2$ |
| $NH_2$ | $NH_2$ | O-phenyl |
| $NH_2$ | $NH_2$ | O-(4-Cl-phenyl) |
| $NH_2$ | $CF_3(C{=}O)NH$ | O- (3-$CF_3$-phenyl) |
| $NH_2$ | $CF_3(C{=}O)NH$ | H |
| $NH_2$ | $CF_3(C{=}O)NH$ | F |
| $NH_2$ | $CF_3(C{=}O)NH$ | Cl |

(fortgesetzt)

| $R^2$ | $R^3$ | $R^4$ |
|---|---|---|
| $NH_2$ | $CF_3(C{=}O)NH$ | CN |
| $NH_2$ | $CF_3(C{=}O)NH$ | $CH_3$ |
| $NH_2$ | $CF_3(C{=}O)NH$ | $CH_3O$ |
| $NH_2$ | $CF_3(C{=}O)NH$ | $C_2H_5S$ |
| $NH_2$ | $CF_3(C{=}O)NH$ | $CH_3S$ |
| $NH_2$ | $CF_3(C{=}O)NH$ | $C_2H_5S$ |
| $NH_2$ | $CF_3(C{=}O)NH$ | benzyl |
| $NH_2$ | $CF_3(C{=}O)NH$ | $CF{=}CF_2$ |
| $NH_2$ | $CF_3(C{=}O)NH$ | $C_2F_5$ |
| $NH_2$ | $CF_3(C{=}O)NH$ | cyclopropyl |
| $NH_2$ | $CF_3(C{=}O)NH$ | $C_2H_5$ |
| $NH_2$ | $CF_3(C{=}O)NH$ | $CF_3O$ |
| $NH_2$ | $CF_3(C{=}O)NH$ | $F_2CHO$ |
| $NH_2$ | $CF_3(C{=}O)NH$ | $C_2F_5O$ |
| $NH_2$ | $CF_3(C{=}O)NH$ | $CF_3S$ |
| $NH_2$ | $CF_3(C{=}O)NH$ | $F_2CHS$ |
| $NH_2$ | $CF_3(C{=}O)NH$ | $CF_3$ |
| $NH_2$ | $CF_3(C{=}O)NH$ | $CH_3SO$ |
| $NH_2$ | $CF_3(C{=}O)NH$ | $CH_3SO_2$ |
| $NH_2$ | $CF_3(C{=}O)NH$ | $CF_3SO_2$ |
| $NH_2$ | $CF_3(C{=}O)NH$ | O-phenyl |
| $NH_2$ | $CF_3(C{=}O)NH$ | O-(4-Cl-phenyl) |
| $CF_3(C{=}O)NH$ | Cl | O-(3-$CF_3$-Phenyl) |
| $CF_3(C{=}O)NH$ | Cl | H |
| $CF_3(C{=}O)NH$ | Cl | F |
| $CF_3(C{=}O)NH$ | Cl | Cl |
| $CF_3(C{=}O)NH$ | Cl | CN |
| $CF_3(C{=}O)NH$ | Cl | $CH_3$ |
| $CF_3(C{=}O)NH$ | Cl | $CH_3O$ |

| $R^2$ | $R^3$ | $R^4$ |
|---|---|---|
| $CF_3(C{=}O)NH$ | Cl | $C_2H_5O$ |
| $CF_3(C{=}O)NH$ | Cl | $CH_3S$ |
| $CF_3(C{=}O)NH$ | Cl | $C_2H_5S$ |
| $CF_3(C{=}O)NH$ | Cl | benzyl |
| $CF_3(C{=}O)NH$ | Cl | $CF{=}CF_2$ |
| $CF_3(C{=}O)NH$ | Cl | $C_2F_5$ |

(fortgesetzt)

| $R^2$ | $R^3$ | $R^4$ |
|---|---|---|
| $CF_3(C{=}O)NH$ | Cl | cyclopropyl |
| $CF_3(C{=}O)NH$ | Cl | $C_2H_5$ |
| $CF_3(C{=}O)NH$ | Cl | $CF_3O$ |
| $CF_3(C{=}O)NH$ | Cl | $F_2CHO$ |
| $CF_3(C{=}O)NH$ | Cl | $C_2F_5O$ |
| $CF_3(C{=}O)NH$ | Cl | $CF_3S$ |
| $CF_3(C{=}O)NH$ | Cl | $F_2CHS$ |
| $CF_3(C{=}O)NH$ | Cl | $CF_3$ |
| $CF_3(C{=}O)NH$ | Cl | $CH_3SO$ |
| $CF_3(C{=}O)NH$ | Cl | $CH_3SO_2$ |
| $CF_3(C{=}O)NH$ | Cl | $CF_3SO_2$ |
| $CF_3(C{=}O)NH$ | Cl | O-phenyl |
| $CF_3(C{=}O)NH$ | Cl | O-(4-Cl-phenyl) |
| $CF_3(C{=}O)NH$ | $CH_3$ | O-(3-$CF_3$-Phenyl) |
| $CF_3(C{=}O)NH$ | $CH_3$ | H |
| $CF_3(C{=}O)NH$ | $CH_3$ | F |
| $CF_3(C{=}O)NH$ | $CH_3$ | Cl |
| $CF_3(C{=}O)NH$ | $CH_3$ | CN |
| $CF_3(C{=}O)NH$ | $CH_3$ | $CH_3$ |
| $CF_3(C{=}O)NH$ | $CH_3$ | $CH_3O$ |
| $CF_3(C{=}O)NHO$ | $CH_3$ | $C_2H_5$ |
| $CF_3(C{=}O)NH$ | $CH_3$ | $CH_3S$ |
| $CF_3(C{=}O)NH$ | $CH_3$ | $C_2H_5S$ |
| $CF_3(C{=}O)NH$ | $CH_3$ | benzyl |
| $CF_3(C{=}O)NH$ | $CH_3$ | $CF{=}CF_2$ |
| $CF_3(C{=}O)NH$ | $CH_3$ | $C_2F_5$ |
| $CF_3(C{=}O)NH$ | $CH_3$ | cyclopropyl |
| $CF_3(C{=}O)NH$ | $CH_3$ | $C_2H_5$ |
| $CF_3(C{=}O)NH$ | $CH_3$ | $CF_3O$ |
| $CF_3(C{=}O)NH$ | $CH_3$ | $F_2CHO$ |
| $CF_3(C{=}O)NH$ | $CH_3$ | $C_2F_5O$ |
| $CF_3(C{=}O)NH$ | $CH_3$ | $CF_3S$ |
| $CF_3(C{=}O)NH$ | $CH_3$ | $F_2CHS$ |

| $R^2$ | $R^3$ | $R^4$ |
|---|---|---|
| $CF_3(C{=}O)NH$ | $CH_3$ | $CF_3$ |
| $CF_3(C{=}O)NH$ | $CH_3$ | $CH_3SO$ |

(fortgesetzt)

| $R^2$ | $R^3$ | $R^4$ |
|---|---|---|
| $CF_3(C=O)NH$ | $CH_3$ | $CH_3SO_2$ |
| $CF_3(C=O)NH$ | $CH_3$ | $CF_3SO_2$ |
| $CF_3(C=O)NH$ | $CH_3$ | O-phenyl |
| $CF_3(C=O)NH$ | $CH_3$ | O-(4-Cl-phenyl) |
| $CF_3(C=O)NH$ | $CH_3O$ | O-(3-$CF_3$-phenyl) |
| $CF_3(C=O)NH$ | $CH_3O$ | H |
| $CF_3(C=O)NH$ | $CH_3O$ | F |
| $CF_3(C=O)NH$ | $CH_3O$ | Cl |
| $CF_3(C=O)NH$ | $CH_3O$ | CN |
| $CF_3(C=O)NH$ | $CH_3O$ | $CH_3$ |
| $CF_3(C=O)NH$ | $CH_3O$ | $CH_3O$ |
| $CF_3(C=O)NH$ | $CH_3O$ | $C_2H_5O$ |
| $CF_3(C=O)NH$ | $CH_3O$ | $CH_3S$ |
| $CF_3(C=O)NH$ | $CH_3O$ | $C_2H_5S$ |
| $CF_3(C=O)NH$ | $CH_3O$ | benzyl |
| $CF_3(C=O)NH$ | $CH_3O$ | $CF=CF_2$ |
| $CF_3(C=O)NH$ | $CH_3O$ | $C_2F_5$ |
| $CF_3(C=O)NH$ | $CH_3O$ | cyclopropyl |
| $CF_3(C=O)NH$ | $CH_3O$ | $C_2H_5$ |
| $CF_3(C=O)NH$ | $CH_3O$ | $CF_3O$ |
| $CF_3(C=O)NH$ | $CH_3O$ | $F_2CHO$ |
| $CF_3(C=O)NH$ | $CH_3O$ | $C_2F_5O$ |
| $CF_3(C=O)NH$ | $CH_3O$ | $CF_3S$ |
| $CF_3(C=O)NH$ | $CH_3O$ | $F_2CHS$ |
| $CF_3(C=O)NH$ | $CH_3O$ | $CF_3$ |
| $CF_3(C=O)NH$ | $CH_3O$ | $CH_3SO$ |
| $CF_3(C=O)NH$ | $CH_3O$ | $CH_3SO_2$ |
| $CF_3(C=O)NH$ | $CH_3O$ | $CF_3SO_2$ |
| $CF_3(C=O)NH$ | $CH_3O$ | O-phenyl |
| $CF_3(C=O)NH$ | $CH_3O$ | O-(4-Cl-phenyl) |

| $R^2$ | $R^3$ | $R^4$ |
|---|---|---|
| Cl | Cl | $NH_2$ |
| $CH_3$ | Cl | $NH_2$ |
| Cl | $CH_3$ | $NH_2$ |
| $CH_3$ | $CH_3$ | $NH_2$ |
| $CH_3O$ | Cl | $NH_2$ |

(fortgesetzt)

| $R^2$ | $R^3$ | $R^4$ |
|---|---|---|
| Cl | $CH_3O$ | $NH_2$ |
| Cl | Cl | $CF_3(C{=}O)NH$ |
| $CH_3$ | Cl | $CF_3(C{=}O)NH$ |
| Cl | $CH_3$ | $CF_3(C{=}O)NH$ |
| $CH_3O$ | Cl | $CF_3(C{=}O)NH$ |
| Cl | $CH_3O$ | $CF_3(C{=}O)NH$ |
| $C_6H_5CH_2NH$ | H | H |
| $C_6H_5CH_2NH$ | Cl | H |

| $R^2$ | $R^3$ | $R^4$ |
|---|---|---|
| $CH_3$ | Cl | $C_6H_5CH_2NH$ |
| $CH_3O$ | Cl | $C_6H_5CH_2NH$ |

Bevorzugte Definitionen für $R^1$ sind:

| Nr. | $R^1$ | Nr. | $R^1$ |
|---|---|---|---|
| L1 | $OCH_3$ | L34 | $-C(CH_3)_2-C_2H_5$ |
| L2 | $OC_2H_5$ | L35 | $-C(CH_3,C_2H_5)C_2H_5$ |
| L3 | $O-i-C_3H_7$ | L36 | $-C(CH_3)_2C_3H_7$ |
| L4 | $n-C_3H_7$ | L37 | $-C(CH_3)_2cycloC_6C_{11}$ |
| L5 | $i-C_3H_7$ | L38 | $-CH_2-C(CH_3)=CH_2$ |
| L6 | $n-C_4H_9$ | L39 | $-CH_2CH=CHCH_3$ |
| L7 | $i-C_4H_9$ | L40 | $-CH(CH_3)CH=CHCH_3$ |
| L8 | $sec-C_4H_9$ | L41 | $-C(CH_3)_2CH=CHCH_3$ |
| L9 | $tert.-C_4H_9$ | L42 | $-CH_2C\equiv CH$ |
| L10 | $n-C_5H_{11}$ | L43 | $-CH(CH_3)C\equiv CH$ |
| L11 | $-CH(CH_3)C_3H_7$ | L44 | $-C(CH_3)_2C\equiv CH$ |
| L12 | $-CH(C_2H_5)C_2H_5$ | L45 | $-C(CH_3,C_2H_5)C\equiv CH$ |
| L13 | $n-C_6H_{13}$ | L46 | $-C(C_2H_5)_2C\equiv CH$ |
| L14 | $-CH(CH_3)C_4H_9$ | L47 | $-CH_2C\equiv CCH_3$ |
| L15 | $-CH(C_2H_5)C_3H_7$ | L48 | $-CH(CH_3)C\equiv CCH_3$ |
| L16 | $OC(CH_3)_3$ | L49 | $-C(CH_3)_2C\equiv CCH_3$ |
| L17 | $cyclo-C_3H_5$ | L50 | $-CH(CH_3)CH_2SCH_3$ |
| L18 | $cyclo-C_4H_7$ | L51 | $-C(CH_3)_2CH_2SCH_3$ |
| L19 | $cyclo-C_5H_9$ | L52 | $-CH_2CH_2CH_2SCH_3$ |
| L20 | $cyclo-C_6H_{11}$ | L53 | $-CH(CH_3)CH_2Cl$ |
| L21 | $cyclo-C_7H_{13}$ | L54 | $-C(CH_3)_2CH_2Cl$ |
| L22 | $cyclo-C_8H_{15}$ | L55 | $-CH(CH_3)CH_2OCH_3$ |

(fortgesetzt)

| Nr. | $R^1$ | Nr. | $R^1$ |
|---|---|---|---|
| L23 | 1-Methylcyclohexyl | L56 | $-C(CH_3)_2CH_2OCH_3$ |
| L24 | 1-Ethylcyclohexyl | L57 | $-CH_2CH_2CH_2OCH_3$ |
| L25 | 3,5-Dimethylcyclohexyl | L58 | $-CH_2CH_2CH_2N(CH_3)_2$ |
| L26 | 3-Trifluormethylcyclohexyl | L59 | $-CH_2CH_2CH_2N(C_2H_5)_2$ |
| L27 | 1-(Cyclopropyl)ethyl | L60 | Cyclopropylmethyl |
| L28 | 1-(Cyclopentyl)ethyl | L61 | $C(CH_3)_2CH_2F$ |
| L29 | 1-(Cyclohexyl)ethyl | L62 | H |
| L30 | $-CH_2-CH=CH_2$ | L63 | $CH_3$ |
| L31 | $-CH(CH_3)CH=CH_2$ | L64 | $C_2H_5$ |

| Nr. | $R^1$ | Nr. | $R^4$ |
|---|---|---|---|
| L32 | $-C(CH_3)_2CH=CH_2$ | L65 | 1-Methylcyclopropyl |
| L33 | $-C(CH_3,C_2H_5)CH=CH_2$ | L66 | 1-Ethylcyclopropyl |

Die Verbindungen I bzw. die sie enthaltenden herbiziden Mittel sowie deren umweltverträgliche Salze z.B. von Alkalimetallen und Erdalkalimetallen können in Kulturen wie Weizen, Reis, Mais, Soja und Baumwolle Unkräuter und Schadgräser sehr gut bekämpfen, ohne die Kulturpflanzen zu schädigen, ein Effekt, der vor allem auch bei niedrigen Aufwandmengen auftritt.

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Methanol, Ethanol, Propanol, Butanol, Cyclohexanol, Cyclohexanon oder stark polare Lösungsmittel, wie N-Methylpyrrolidon oder Wasser in Betracht.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z. B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an

feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Formulierungen enthalten im allgemeinen zwischen 0,01 bis 95 Gew.-%, vorzugsweise zwischen 0,5 bis 90 Gew.-%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können weiterhin beispielsweise wie folgt formuliert werden:

I. 20 Gew.-Teile der Verbindung Nr. 1.001 werden in einer Mischung gelöst, die aus 80 Gew.-Teilen alkyliertem Benzol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in 100 000 Gew.-Teilen Wasser erhält man eine wäßrige Dispersion, die 0,02 Gew.-% des Wirkstoffs enthält.

II. 20 Gew.-Teile der Verbindung Nr. 1.004 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenyl und 10 Gew.-Teilen des Anlagerungsproduktes 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.

III. 20 Gew.-Teile des Wirkstoffs Nr. 1.006 werden in einer Mischung gelöst, die aus 25 Gew.-Teilen Cyclohexanon, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinus besteht. Durch Eingießen und feines Verteilen der Lösung in 100 000 Gew.-Teilen Wasser erhält man eine wäßrige Dispersion, die 0,02 Gew.-% des Wirkstoffs enthält.

IV. 20 Gew.-Teile des Wirkstoffs Nr. 1.016 werden mit 3 Gew.-Teilen des Natriumsalzes der Diisobutylnaphthalinα-sulfonsäure, 17 Gew.-Teile des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser enthält man eine Spritzbrühe, die 0,1 Gew.-% des Wirkstoffs enthält.

V. 3 Gew.-Teile des Wirkstoffs Nr. 1.018 werden mit 97 Gew.-Teilen feinteiligem Kaolin vermischt. Man erhält auf diese Weise ein Stäubemittel, das 3 Gew.-% des Wirkstoffs enthält.

VI. 20 Gew.-Teile des Wirkstoffs Nr. 1.023 werden mit 2 Gew.-Teilen Calciumsalz der Dodecylbenzolsulfonsäure, 8 Gew.-Teilen Fettalkohol-polyglykolether, 2 Gew.-Teilen Natriumsalz eines Phenol-Harnstoff-Formaldehyd-Kondensates und 68 Gew.-Teilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,01 bis 5,0, vorzugsweise 0,05 bis 2,0 kg/ha aktive Substanz (a.S.).

In Anbetracht der Vielseitigkeit der Applikationsmethoden können die erfindungsgemäßen Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N. rustica), Olea europaea, Oryza sativa, Phaseolus lunatus, Phaseolus vulgaris, Picea abies, Pinus spp., Pisum sativum,

Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Zur Verbreiterung des Wirkungsspektrums und zur Erzielung synergistischer Effekte können die Pyridin-2,3-dicarbonsäureimide I mit zahlreichen Vertretern anderer herbizider oder wachstumsregulierender Wirkstoffgruppen gemischt und gemeinsam ausgebracht werden. Beispielsweise kommen als herbizide Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazoline, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden oder Wachstumsregulatoren auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

A) Herstellung von Vorprodukten

5-Nitro-pyridin-2,3-dicarbonsäurediethylester

255 ml Essigsäureanhydrid wurden zu einer Mischung von 203,2 g (0,85 mol) Pyridin-2,3-dicarbonsäurediethylester-N-oxid in 1,2 l Eisessig gegeben. Anschließend wurden bei 20 bis 28°C unter leichter Kühlung und Rühren 68,3 g (1,06 mol) 98 %ige Salpetersäure innerhalb 40 Minuten zugeführt und 12 Stunden bei 30°C nachgerührt. Die Reaktionslösung wurde auf 3,5 l Eiswasser gegossen und mit Methylenchlorid extrahiert. Die organische Phase wurde nacheinander mit Wasser, gesättigter Natriumhydrogencarbonatlösung und Kochsalzlösung extrahiert, über Magnesiumsulfat getrocknet und über Kieselgel abgesaugt. Nach dem Einengen erhielt man 177,6 g (78 % d. Th.) des 5-Nitro-pyridin-2,3-dicarbonsäurediethylester-N-oxids mit $n_D^{25}$25 = 1.5290. Eine Probe kristallisierte nach längerem Stehen; Fp. 84-86°C.

88,8 g (0,648 mol) Phosphortrichlorid wurden innerhalb 30 Minuten unter Rühren bei 25°C zu einer Mischung von 92 g (0,324 mol) obigen N-Oxids und 300 ml 1,2-Dichlorethan gegeben. Nach 12 Stunden Rühren bei 25°C wurde das Reaktionsgemisch im Vakuum eingeengt, erneut in Methylenchlorid aufgenommen und auf 2,5 l Eiswasser gegossen. Nach Phasentrennung wurde die wäßrige Phase mit Methylenchlorid extrahiert. Der organische Extrakt wurde mit Wasser, gesättigter Natriumhydrogencarbonat- und Kochsalzlösung gewaschen, über Magnesiumsulfat getrocknet und über neutrales Aluminiumoxid sowie Kieselgel abgesaugt. Nach dem Einengen erhielt man 58,2 g (67 % d. Th.) der Titelverbindung als gelblichen Sirup.

$^1$H-NMR, 400 MHz ($CDCl_3$) δ: Ar 9,0 (d/1H), 9,55 (d,1H)

5-Nitro-pyridin-2,3-dicarbonsäureanhydrid

45 g (0,168 mol) 5-Nitropyridin-2,3-dicarbonsäurediethylester wurden innerhalb 10 Minuten unter Rühren zu einer Mischung von 13,4 g (0,336 mol) Natriumhydroxid in 65 ml Wasser gegeben und mit 6,5 ml Ethanol nachgewaschen. Die Mischung wurde zum Rückfluß gebracht, mit weiteren 30 ml Wasser verdünnt und nach 1 Stunde Erhitzen wieder abgekühlt. Der nach der Zugabe von 500 ml Aceton ausgefällte Niederschlag wurde abgesaugt, gewaschen und getrocknet, wobei man 42,4 g (98,6 % d. Th.) 5-Nitro-pyridin-2,3-dicarbonsäure-natriumsalz mit Fp. > 260°C erhielt.

Dieses wurde in 500 ml 1,2-Dichlorethan vorgelegt und innerhalb 15 Minuten mit 39 g (0,497 mol) Acetylchlorid vereinigt. Das Reaktionsgemisch wurde 4 Stunden unter Rückfluß gerührt, abgekühlt, vom ausgefallenen Niederschlag abgetrennt und mit Methylenchlorid gewaschen. Nach dem Einengen im Vakuum, Verreiben des Rückstands mit Ether/Pentan, Absaugen und Trocknen erhielt man 25,4 g (79 % d. Th.) der Titelverbindung vom Fp. 85-91°C.

5-Amino-pyridin-2,3-dicarbonsäurediethylester

17 g (0,060 mol) 5-Nitro-pyridin-2,3-dicarbonsäurediethylester-N-oxid in 200 ml Eisessig wurden bei 70°C unter Rühren innerhalb 20 Minuten zu einer Mischung von 10,7 g (0,19 mol) Eisenpulver in 50 ml Eisessig gegeben. Nach 1 Stunde Rühren bei 70°C wurde das Reaktionsgemisch abgekühlt, der Niederschlag abgesaugt und mit Essigester gewaschen. Das Filtrat wurde im Vakuum eingeengt, in Methylenchlorid gelöst, mit Wasser und mit gesättigter Kochsalzlösung gewaschen, über Magnesiumsulfat getrocknet und über Kieselgel abgesaugt. Nach dem Einengen im Vakuum erhielt man 11 g (77 % d. Th.) der Titelverbindung als eine halbfeste Masse.

1H-NMR, 270 MHz ($CDCl_3$) δ: Ar 7,08 (d/1), 8,15 (d,1), $NH^2$ 4,45 (s/2)

6-Chlor-5-nitro-pyridin-2,3-dicarbonsaurediethylester

100 g (0,352 mol) 5-Nitro-pyridin-2,3-dicarbonsäurediethylester wurden innerhalb 15 Minuten unter Rühren bei 60°C zu 500 ml Phosphorylchlorid gegeben, zum Rückfluß erwärmt und 1 1/2 Stunden nachgerührt. Nach dem

Abkühlen wurde das Reaktionsgemisch im Vakuum eingeengt und wieder in Methylenchlorid gelöst. Nach Verrühren mit Eiswasser wurde die wäßrige Phase nochmals mit Methylenchlorid gewaschen und dann die organischen Auszüge nacheinander mit Wasser, gesättigter Natriumhydrogencarbonat- und Kochsalzlösung gewaschen und über Magnesiumsulfat getrocknet. Nach dem Absaugen über neutrales Aluminiumoxid und Einengen erhielt man 68,8 g (64,6 % d. Th.) der Titelverbindung mit Fp. 70-72°C. (erfindungsgemäß nicht beansprucht)

6-Benzylamino-pyridin-2,3-dicarbonsäurediethylester

10,7 g (0,1 mol) Benzylamin und 10,1 g (0,1 mol) Triethylamin wurden innerhalb 10 Minuten unter Rühren zu einer Mischung von 25,8 g (0,1 mol) 6-Chlor-pyridin-2,3-dicarbonsäurediethylester in 250 ml Propanol bei 25°C gegeben. Nach 12 Stunden Rühren unter Rückfluß wurde das Reaktionsgemisch eingeengt, in Methylenchlorid aufgenommen und mit Wasser extrahiert. Die organische Phase wurde getrocknet, über neutrales Aluminiumoxid gesaugt und im Vakuum eingeengt. Der Rückstand wurde in Ether/Pentan verrührt, abgesaugt und getrocknet, wobei man 23,3 g (71,1 % d. Th.) der Titelverbindung vom Fp. 110-114°C erhielt.

5-Trifluoracetamino-pyridin-2,3-dicarbonsäurediethylester

23,8 g (0,114 mol) Trifluoressigsäureanhydrid wurden innerhalb 10 Minuten unter Rühren bei 25°C zu einer Mischung von 10,8 g (0,045 mol) 5-Aminopyridin-2,3-dicarbonsäurediethylester in 100 ml 1,2-Dichlorethan gegeben und 3 Stunden unter Rückfluß gekocht. Das Reaktionsgemisch wurde in 500 ml Eiswasser eingetragen und dreimal mit Methylenchlorid extrahiert. Der organische Extrakt wurde mit Wasser, gesättigter Natriumhydrogencarbonat- sowie einer Kochsalzlösung gewaschen und über Magnesiumsulfat getrocknet. Nach dem Einengen im Vakuum erhielt man 14,6 g (96 % d. Th.) der Titelverbindung als farblosen Sirup mit $n_D^{25}$ = 1.5018.

$^1$H-NMR, 200 MHz ($CDCl_3$) δ: Ar 8,63 (s/1), 8,95 (s,1), NH 11,25 (s/1)

B) Herstellung der Endprodukte I

5-Nitro-pyridin-2,3-dicarbonsäure-N-1-(cyclopropyl)ethylimid

11,2 g (0,132 mol) 1-Cyclopropylethylamin wurden bei 25°C unter Rühren innerhalb 8 Minuten zu einer Mischung von 22,3 g (0,115 mol) 5-Nitropyridin-2,3-dicarbonsäureanhydrid in 150 ml 1,2-Dichlorethan gegeben und 3 Stunden bei 70°C gerührt. Nach dem Abkühlen auf 25°C wurden 19,6 g (0,165 mol) Thionylchlorid unter Rühren innerhalb 10 Minuten zugegeben und 10 Stunden bei 25°C und 1 1/2 Stunden bei 70°C gerührt. Das Reaktionsgemisch wurde mit 100 ml Methylenchlorid verdünnt und unter Rühren auf 500 ml Eiswasser gegeben. Die wäßrige Phase wurde noch zweimal mit Methylenchlorid extrahiert. Anschließend wurde die organische Phase nacheinander mit Wasser, gesättiger Natriumhydrogencarbonat- und mit Kochsalzlösung und über neutrales Aluminiumoxid gesaugt. Nach dem Einengen erhielt man 21,5 g (72 % d. Th.) der Titelverbindung mit Fp. 96-99°C (Wirkstoffbeispiel 1.020).

5-Nitro-pyridin-2,3-dicarbonsäure-N-tert.-butylimid

Zu einer Suspension von 53,6 g (0,244 mol)

Pyridin-2,3-dicarbonsäure-N-tert-butylimid-1-oxid in 150 ml Eisessig und 71,4 g (0,7 mol) Essigsäureanhydrid wurden innerhalb 10 Minuten die Hälfte von 18 g (0,28 mol) 98 %ige Salpetersäure unter Rühren bei 20 bis 25°C und nach 1 Stunde der Rest zugegeben und 16 Stunden bei 25°C gerührt. Das Reaktionsgemisch wurde auf 1,5 l Eiswasser gegossen und dreimal mit je 300 ml Methylenchlorid extrahiert. Der organische Extrakt wurde nacheinander mit Wasser, gesättigter Natriumhydrogencarbonat- und Kochsalzlösung gewaschen und über Magnesiumsulfat getrocknet. Nach dem Einengen im Vakuum erhielt man 55 g eines gelben Sirups. Dieser wurde über Kieselgel mit Methylenchlorid flash-chromatographiert und die mittlere Fraktion eingeengt (28 g).

Diese wurden in 150 ml 1,2-Dichlorethan vorgelegt mit 28,9 g (0,213 mol) Phosphortrichlorid unter Rühren innerhalb 10 Minuten bei 25°C versetzt und 1 1/2 Stunden bei 75°C gerührt. Die Reaktionsmischung wurde im Vakuum eingeengt, in Methylenchlorid aufgenommen und in Eiswasser eingerührt. Die organische Phase wurde nacheinander mit Wasser, gesättigter Natriumhydrogencarbonat- und mit Kochsalzlösung extrahiert und über Magnesiumsulfat getrocknet. Nach dem Absaugen über neutrales Aluminiumoxid wurde eingeengt, der Rückstand mit Ether/Pentan verrührt, abgesaugt und getrocknet. Man erhielt 20,5 g (33,7 % d. Th.) der Titelverbindung vom Fp. 170-173°C (Wirkstoffbeispiel 1.021).

6-Chlor-5-nitro-pyridin-2,3-dicarbonsäure-N-tert.-butylimid

100 ml Phosphorylchlorid wurden bei 60°C und portionsweise innerhalb 2 Minuten unter Rühren mit 7,5 g (0,028 mol) 5-Nitro-pyridin-2,3-dicarbonsäure-N-tert.-butylimid-1-oxid versetzt und 2 Stunden unter Rückfluß gerührt. Das Reaktionsgemisch wurde im Vakuum eingeengt, in Methylenchlorid aufgenommen und in Eiswasser eingerührt. Die organische Phase wurde nacheinander mit Wasser, gesättigter Natriumhydrogencarbonat- und Kochsalzlösung extrahiert und getrocknet. Nach dem Absaugen über neutrales Aluminiumoxid wurde eingeengt und der Rückstand mit Ether sowie Ether/Pentan verrührt, abgesaugt und getrocknet. Man erhielt 4,49 g (56 % d. Th.) der Titelverbindung vom Fp. 100-104°C (Wirkstoffbeispiel 1.019).

6-Amino-5-methyl-pyridin-2,3-dicarbonsäure-N-1-(cyclopropyl)ethylimid

6,3 g (0,0254 mol) 6-Fluor-5-methyl-pyridin-2,3-dicarbonsäure-N-1-(cyclopropyl)ethylimid und 4,1 g (0,051

mol) Kaliumcyanat wurden bei 25°C unter Rühren in eine Mischung von 50 ml DMF und 1 ml Wasser eingetragen und dann 30 Minuten bei 125°C gerührt. Nach dem Einengen der Reaktionsmischung im Vakuum wurde der Rückstand nacheinander mit Methyl-tert.- butylether und mit Wasser verrührt. Unlösliches wurde abgesaugt, mit Methyl-tert.-butylether gewaschen und im Vakuum getrocknet. Man erhielt 3,5 g (56 % d.Th.) der Titelverbindung vom Fp. 219-220°C. Aus der organischen Phase erhielt man nach dem Waschen mit Wasser und Trocknen über Magnesiumsulfat weitere 1,6 g (=26 % d. Th.) der Titelverbindung vom Fp. 201-219°C (Wirkstoffbeispiel 1.059).

4-Amino-6-chlor-pyridin- und 6-Amino-4-chlorpyridin-2,3-dicarbonsäure-N-tert.-butylimid

14.3 g (0,052 mol) 4,6-Dichlorpyridin-2,3-dicarbonsäure-N-tert.-butylimid und 8,5 g (0,105 mol) Kaliumcyanat wurden bei 25°C in eine Mischung von 100 ml DMF und 2 ml Wasser gegeben und 20 Minuten bei 125°C gerührt.

Nach dem Einengen der Reaktionsmischung im Vakuum wurde der Rückstand in einer Mischung von Methyl-tert.-butylether und Wasser verrührt und abgesaugt. Nochmaliges Verrühren mit Methyl-tert.-butylether, Absaugen und Trocknen lieferte 8,73 g (66 % d. Th.) 4-Amino-6-chlor-pyridin-2,3-dicarbonsäure-N-tert.-butylimid vom Fp. 232-234°C (Wirkstoffbeispiel 1.048).

Das organische Filtrat wurde mit Wasser und gesättigter Kochsalzlösung gewaschen, über Magnesiumsulfat getrocknet und eingeengt. Der Rückstand wurde in Methylenchlorid aufgenommen und über Kieselgel abgesaugt, wobei man aus den Fraktionen 3 und 4 0,95 g (7,2 % d. Th.) 6-Amino-4-chlor-pyridin-2,3-dicarbonsäure-N-tert.-butylimid vom Fp. 215-220°C isolierte (Wirkstoffbeispiel 1.053).

Entsprechend diesen in den Beispielen B sowie den Vorprodukten beschriebenen Verfahren wurden die in der Tabelle 1 aufgelisteten Pyridin-2,3-dicarbonsäureimide der Formel I erhalten. In den Tabellen 2 und 3 sind neue Pyridin-2,3-dicarbonsäureanhydride bzw. Pyridin-2,3-dicarbonsäureester aufgeführt.

Tabelle 1

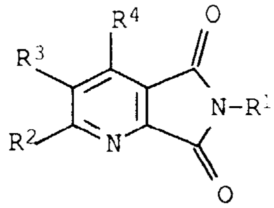

I

| Nr. | $R^1$ | $R^2$ | $R^3$ | $R^4$ | Fp. (°C) |
|---|---|---|---|---|---|
| 1.001 | tert.-$C_4$-$H_9$ | $CH_3$ | $NO_2$ | H | 118-119 |
| 1.002 | i-$C_3H_7$ | $CH_3$ | $NO_2$ | H | 90- 91 |
| 1.003 | tert.-$C_4H_9$ | $CH_3$ | O<br>‖<br>$CH_3$-C-NH | H | 162-163 |
| 1.004 | tert.-$C_4H_9$ | $CH_3$ | O<br>‖<br>$CF_3$-C-NH | H | 68- 71 |
| 1.005 | tert.-$C_4H_9$ | $CH_3$ | S=C=N- | H | 94- 95 |
| 1.006 | tert.-$C_4H_9$ | $CH_3$ | $NH_2$ | H | 242 |
| 1.007 | tert.-$C_4H_9$ | Cl | $NH_2$ | Cl | |
| 1.008 | i-$C_3H_7$ | $CH_3$ | O<br>‖<br>$CF_3$-C-NH | H | 116-117 |
| 1.009 | i-$C_3H_7$ | $CH_3$ | $NH_2$ | H | 217-221 |
| 1.010 | i-$C_3H_7$ | $CH_3$ | S=C=N- | H | 78- 80 |
| 1.011 | i-$C_3H_7$ | $CH_3$ | O<br>‖<br>$CH_3$-C-NH | H | 160-161 |
| 1.012 | 1-(Cyclopropyl)ethyl | $CH_3$ | S=C=N | H | 98-100 |
| 1.013 | 1-(Cyclopropyl)ethyl | $CH_3$ | O<br>‖<br>$CF_3$-C-NH | H | 64- 65 |
| 1.014 | 1-(Cyclopropyl)ethyl | $CH_3$ | O<br>‖<br>$CH_3$-C-NH | H | 178-179 |
| 1.015 | 1-(Cyclopropyl)ethyl | $CH_3$ | $NH_2$ | H | 170-171 |
| 1.016 | 1-(Cyclopropyl)ethyl | $CH_3$ | $NO_2$ | H | $n_D^{23}$=1.5670 |

| Nr. | $R^1$ | $R^2$ | $R^3$ | $R^4$ | Fp. (°C) |
|---|---|---|---|---|---|
| 1.017 | i-$C_3H_7$ | $CH_3$ | $CH_3SO_2NH$ | H | 158-160 |
| 1.018 | tert.-$C_4H_9$ | $CH_3$ | $CH_3SO_2NH$ | H | 149-153 |
| 1.019 | tert.-$C_4H_9$ | Cl | $NO_2$ | H | 100-104 |
| 1.020 | 1-(Cyclopropyl)ethyl | H | $NO_2$ | H | 96- 99 |
| 1.021 | tert.-$C_4H_9$ | H | $NO_2$ | H | 170-173 |
| 1.022 | i-$C_3H_7$ | H | $NO_2$ | H | 166-169 |
| 1.023 | 1-(Cyclopropyl)ethyl | Cl | $NO_2$ | H | 65- 66 |
| 1.024 | tert.-$C_4H_9$ | H | 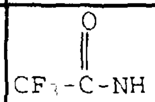 | H | 142-145 |
| 1.025 | 1-(Cyclopropyl)ethyl | $C_6H_5CH_2NH$ | H | H | 144-148 |
| 1.026 | tert.-$C_4H_9$ | $CH_3$ | $CH_3$ | $NH_2$ | |
| 1.027 | 1-(Cyclopropyl)ethyl | $CH_3$ | $CH_3$ | $NH_2$ | |
| 1.030 | tert.-$C_4H_9$ | Cl | $CH_3$ | $NH_2$ | |
| 1.031 | 1-(Cyclopropyl)ethyl | $CH_3$ | Cl | $NH_2$ | |
| 1.032 | tert.-$C_4H_9$ | $CH_3O$ | Cl | $NH_2$ | |
| 1.033 | 1-(Cyclopropyl)ethyl | $CH_3O$ | Cl | $NH_2$ | |
| 1.036 | tert.-$C_4H_9$ | Cl | Cl | $NH_2$ | 232-234 |
| 1.037 | 1-(Cyclopropyl)ethyl | Cl | Cl | $NH_2$ | |
| 1.038 | tert.-$C_4H_9$ | Cl | Cl | $CF_3(C=O)NH$ | |
| 1.039 | tert.-$C_4H_9$ | 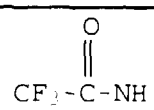 | $CH_3$ | H | 159-160 |
| 1.040 | tert.-$C_4H_9$ | $NH_2$ | $CH_3$ | H | 286-290 |
| 1.041 | tert.-$C_4H_9$ | $NH_2$ | $CH_3O$ | H | 220-225 |
| 1.042 | tert.-$C_4H_9$ | 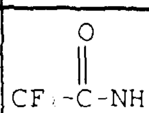 | H | H | 151-154 |
| 1.043 | tert.-$C_4H_9$ | H | 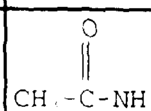 | H | 193-195 |
| 1.044 | tert.-$C_4H_9$ | H | $NH_2$ | H | 187-190 |
| 1.045 | tert.-$C_4H_9$ | 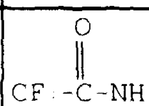 | Cl | H | 168-171 |
| 1.046 | tert.-$C_4H_9$ | $NH_2$ | Cl | H | 258-260 |

| Nr. | $R^1$ | $R^2$ | $R^3$ | $R^4$ | Fp. (°C) |
|---|---|---|---|---|---|
| 1.047 | i-$C_3H_7$ | Cl | Cl | $NH_2$ | 249-253 |
| 1.048 | tert.-$C_4H_9$ | Cl | H | $NH_2$ | 232-234 |
| 1.049 | tert.-$C_4H_9$ | $NH_2$ | Cl | Cl | 215-220 |
| 1.050 | tert.-$C_4H_9$ | 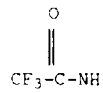 | $CH_3O$ | H | 245-248 |
| 1.051 | i-$C_3H_7$ | Cl | 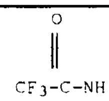 | H | 145-148 |
| 1.052 | tert.-$C_4H_9$ | Cl | 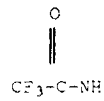 | H | 124-127 |
| 1.053 | tert.-$C_4H_9$ | $NH_2$ | H | Cl | 215-220 |
| 1.054 | tert.-$C_4H_9$ | Cl | Cl | 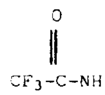 | 135-137 |
| 1.055 | tert.-$C_4H_9$ | Cl | H | 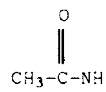 | 136-139 |
| 1.056 | tert.-$C_4H_9$ | Cl | H | 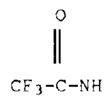 | 105-106 |
| 1.057 | tert.-$C_4H_9$ | Cl | $CH_3O$ | $NH_2$ | 212-215 |
| 1.058 | 1-(Cyclopropyl)ethyl | 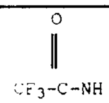 | $CH_3$ | H | 170-172 |
| 1.059 | 1-(Cyclopropyl)ethyl | $NH_2$ | $CH_3$ | H | 219-220 |
| 1.060 | i-$C_3H_7$ | $CH_3$ | 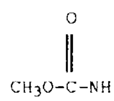 | H | 174-175 |
| 1.061 | tert.-$C_4H_9$ | $CH_3O$ | 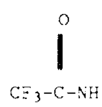 | H | 185-187 |
| 1.062 | tert.-$C_4H_9$ | $CH_3O$ | 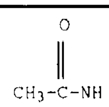 | H | >280 |
| 1.063 | tert.-$C_4H_9$ | $CH_3O$ | $NH_2$ | H | 200-202 |
| 1.064 | tert.-$C_4H_9$ | $CH_3O$ | $NO_2$ | H | 140-141 |
| 1.065 | tert.-$C_4$-$H_9$ | $NH_2$ | H | H | 179-182 |
| 1.066 | i-$C_3H_7$ | $NH_2$ | H | H | 153-157 |
| 1.067 | i-$C_3H_7$ | $NH_2$ | $CH_3$ | H | 255-260 |

| Nr. | $R^1$ | $R^2$ | $R^3$ | $R^4$ | Fp. (°C) |
|---|---|---|---|---|---|
| 1.068 | i-$C_3H_7$ | $CH_3O$ | $NO_2$ | H | 128-131 |
| 1.069 | 1-(Cyclopropyl)ethyl | $CH_3O$ | $NO_2$ | H | 152-154 |
| 1.070 | tert.-$C_4H_9$ | $CH_3$ | Cl | $NH_2$ | 220-221 |
| 1.071 | i-$C_3H_7$ | $CH_3O$ | $NH_2$ | H | 213-214 |
| 1.072 | 1-(Cyclopropyl)ethyl | $CH_3O$ | $NH_2$ | H | 163-165 |
| 1.073 | 1-(Cyclopropylethyl) | Cl | $CH_3$ | $NH_2$ | 236-238 |
| 1.074 | 1-(Cyclopropylethyl) | $NH_2$ | $CH_3$ | Cl | 234-236 |
| 1.075 | i-$C_3H_7$ | Cl | $CH_3$ | $NH_2$ | >305 |
| 1.076 | i-$C_3H_7$ | $NH_2$ | $CH_3$ | Cl | 298-302 |
| 1.077 | i-$C_3H_7$ | $CH_3O$ | 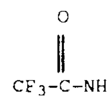 | H | 178-180 |
| 1.078 | 1-(Cyclopropylethyl) | $CH_3O$ | 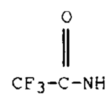 | H | 134-136 |
| 1.079 | 1-(Cyclopropylethyl) | Cl | $CH_3$ | 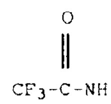 | 162-163 |
| 1.080 | i-$C_3H_7$ | Cl | $CH_3$ | 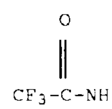 | 155-157 |
| 1.081 | 1-(Cyclopropylethyl) | 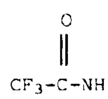 | $CH_3$ | Cl | 81-83 |
| 1.082 | i-$C_3H_7$ | 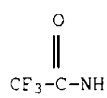 | $CH_3$ | Cl | 111-114 |
| 1.083 | tert.-$C_4H_9$ | Cl | $CH_3$ | $NH_2$ | 253-257 |
| 1.084 | tert.-$C_4H_9$ | $NH_2$ | $CH_3$ | Cl | 215-217 |
| 1.085 | tert.-$C_4H_9$ | Cl | $CH_3$ | 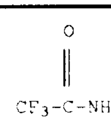 | 172-174 |
| 1.086 | tert.-$C_4H_9$ | 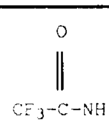 | $CH_3$ | Cl | 85-88 |
| 1.087 | tert.-$C_4H_9$ | Cl | $NH_2$ | H | 240-242 |
| 1.088 | tert.-$C_4H_9$ | Cl | 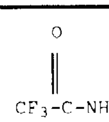 | H | 144-147 |

Tabelle 2

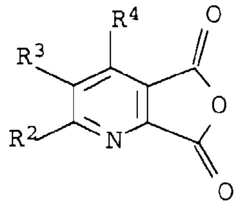

IIa'

| Nr. | $R^2$ | $R^3$ | $R^4$ | physik. Daten - Fp. (°C) $^1H$-NMR, 200 MHz, δ [ppm] in $CDCl_3$ |
|---|---|---|---|---|
| 2.001 | H | $NO_2$ | H | 85-91 |
| 2.002 | Cl | $NO_2$ | H | |
| 2.003 | Cl | $NO_2$ | Cl | |
| 2.004 | $CH_3$ | $NO_2$ | H | $CH_3$: 3,05 (s/3), Ar: 8,05 (s/1) |
| 2.005 | $CH_3$ | $NO_2$ | Cl | |
| 2.006 | H | $NH_2$ | H | |
| 2.007 | H | $CF_3(C=O)NH$ | H | 153-156 |
| 2.008 | $NH_2$ | H | H | |
| 2.009 | $C_6H_5-CH_2NH$ | H | H | |
| 2.010 | 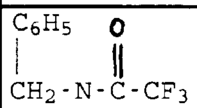 | H | H | 105-107 |
| 2.011 | $CH_3O$ | $NO_2$ | H | 123-127 |
| 2.012 | $CH_3O$ | $NH_2$ | H | |
| 2.013 | $CH_3O$ | $CF_3(C=O)NH$ | H | |
| 2.014 | $CF_3(C=O)NH$ | H | H | |
| 2.015 | Cl | H | $CH_3(C=O)NH$ | |

Tabelle 3

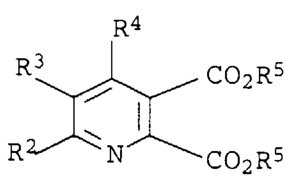

Va'

| Nr. | $R^2$ | $R^3$ | $R^4$ | $R^5$ | physik. Daten - Fp. (°C) $^1$H-NMR, 270 MHz, δ [ppm] in $CDCl_3$ |
|---|---|---|---|---|---|
| 3.001 | H | $NO_2$ | H | $C_2H_5$ | $n_D^{25}$=1.5290 |
| 3.002 | Cl | $NO_2$ | H | $CH_3$ | 68-73 |
| 3.003 | Cl | $NO_2$ | Cl | $C_2H_5$ | |
| 3.004 | $CH_3$ | $NO_2$ | Cl | $CH_3$ | |
| 3.005 | H | $NH_2$ | H | $C_2H_5$ | Ar: 7,08 (d/1), 8,15 (d/1), $NH_2$: 4,45 (s/2) |
| 3.006 | H | $CF_3(C{=}O)NH$ | H | $C_2H_5$ | $n_D^{25}$=1.5018 |
| 3.007 | $NH_2$ | H | H | $CH_3$ | |
| 3.008 | $C_6H_5CH_2NH$ | H | H | $C_2H_5$ | 110-114 |
| 3.009 | $C_6H_5CH_2NH$ | Cl | H | $C_2H_5$ | |
| 3.010 | $CH_3O$ | $NO_2$ | H | $C_2H_5$ | 60-64 |
| 3.011 | $CH_3O$ | $NH_2$ | H | $CH_3$ | |
| 3.012 | $CH_3O$ | $CF_3(C{=}O)NH$ | H | $CH_3$ | |
| 3.013 | Cl | $NH_2$ | H | $C_2H_5$ | |
| 3.014 | NH | Cl | H | $C_2H_5$ | |
| 3.015 | $CH_3O$ | H | $NH_2$ | $CH_3$ | |
| 3.016 | Cl | H | $NH_2$ | $CH_3$ | |
| 3.017 | Cl | $NH_2$ | H | $CH_3$ | 94-100 |
| 3.018 | $CH_3O$ | $NO_2$ | H | $CH_3$ | 57-58 |
| 3.019 | $NH_2$ | H | H | $C_2H_5$ | 149-155 |
| 3.020 | Cl | $NO_2$ | H | $C_2H_5$ | 70-72 |
| 3.021 | Cl | $NO_2$ | H | $CH_3$ | Ar-$CH_3$ 8.78 (s/1) |
| 3.022 | $CH_3$ | $NO_2$ | H | $CH_3$ | 60-64 |

Anwendungsbeispiele

Die herbizide Wirkung der Pyridin-2,3-dicarbonsäureimide der Formel I ließ sich durch Gewächshausversuche zeigen:

Als Kulturgefäße dienten Plastiktöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdekkung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe 3 bis 15 cm angezogen und erst dann mit den in Wasser suspendierten oder emulgierten Wirkstoffen behandelt. Die

Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufanwendung betrug 0,5 bzw. 0,25 kg/ha a.S.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Die herbizide Wirkung wurde nach einer Skala von 0 bis 100 bewertet. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Abkürzung | Lateinischer Name | Deutscher Name |
|---|---|---|
| CHEAL | Chenopodium album | Weißer Gänsefuß |
| POLPE | Polygonum persicaria | Flohknöterich |
| SOLNI | Solanum nigrum | Schwarzer Nachtschatten |

Die Ergebnisse (s. nachfolgende Tabellen I) zeigen die überlegene herbizide Wirkung der erfindungsgemäßen Verbindungen im Vergleich zu dem aus EP-A 422 456 bekannten Vergleichsbeispiel A.

Tabelle I

Beispiele zur Bekämpfung unerwünschter Pflanzen bei Nachauflaufanwendung von 0.5 kg und 0,25 kg a.S./ha im Gewächshaus

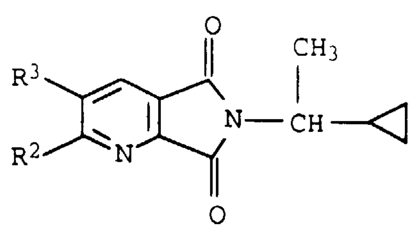

| $R^3$ | $NH_2$ | $NH_2$ | H | H |
|---|---|---|---|---|
| $R^2$ | $CH_3$ | $CH_3$ | $CH_3$ | $CH_3$ |
| Bsp.-Nr. | 1.015 | 1.015 | A | A |
| Aufwandmenge (kg a.S./ha) | 0.5 | 0.25 | 0.5 | 0.25 |
| Testpflanzen | Schädigung in % | | | |
| CHEAL | 100 | 90 | 100 | 75 |
| POLPE | 100 | 100 | 100 | 60 |
| SOLNI | 100 | 100 | 90 | 10 |

## Patentansprüche

**1.** Pyridin-2,3-dicarbonsäureimide der allgemeinen Formel I

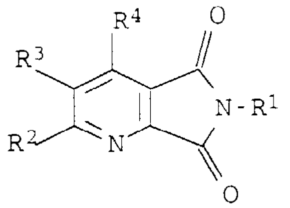

I

in der

$R^1$

Wasserstoff;

$C_1$-$C_4$-Alkoxy;

$C_1$-$C_6$-Alkyl, das eine bis drei der folgenden Gruppen tragen kann: $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Dialkylamino, $C_3$-$C_8$-Cycloalkyl, Halogen, ausgenommen $C_1$-$C_4$-Dialkylaminoethyl, wenn einer der Reste $R^2$, $R^3$ oder $R^4$ Amino oder Hydroxy bedeutet;

$C_3$-$C_8$-Cycloalkyl, das eine bis drei der folgenden Gruppen tragen kann: $C_1$-$C_6$-Alkyl, $C_1$-$C_6$-Halogenalkyl, $C_1$-$C_4$-Alkoxy, $C_1$-$C_4$-Halogenalkoxy, Halogen oder Nitro;

$C_3$-$C_6$-Alkenyl oder $C_3$-$C_6$-Alkinyl, die bis zu dreimal durch Halogen substituiert sein können, bedeutet und mindestens ein Rest $R^2$, $R^3$ oder $R^4$ für eine Gruppe $NR^6R^7$ steht und die übrigen Reste $R^2$, $R^3$ bzw. $R^4$ wie folgt definiert sind:

i) Wasserstoff;

ii) Halogen oder Cyano;

iii) $C_1$-$C_6$-Alkyl, welches durch ein bis fünf Halogenatome und/oder einen bis zwei der folgenden Reste substituiert sein kann: $C_1$-$C_4$-Alkoxy, $C_1$-$C_4$-Halogenalkoxy, $C_1$-$C_4$-Alkylthio, $C_1$-$C_4$-Halogenalkylthio, $C_3$-$C_6$-Cycloalkyl oder Cyano;

iv) Benzyl, das bis zu dreimal durch $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro substituiert sein kann;

v) $C_3$-$C_8$-Cycloalkyl, das ein- bis dreimal durch $C_1$-$C_4$-Alkyl oder Halogen substituiert sein kann;

vi) $C_2$-$C_6$-Alkenyl, welches bis zu dreimal durch Halogen und/oder einmal durch $C_1$-$C_3$-Alkoxy oder durch Phenyl, das eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-halogenalkylthio, Halogen, Cyano oder Nitro, substituiert sein kann;

vii) $C_2$-$C_6$-Alkinyl, welches bis zu dreimal durch Halogen oder $C_1$-$C_3$-Alkoxy und/oder einmal durch Phenyl, das eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro, substituiert sein kann;

viii) $C_1$-$C_4$-Alkoxy, $C_1$-$C_4$-Alkylthio, $C_1$-$C_4$-Halogenalkoxy, $C_1$-$C_4$-Halogenalkylthio, $C_2$-$C_5$-Alkenyloxy, $C_2$-$C_5$-Alkinyloxy, $C_1$-$C_4$-Alkylsulfinyl, $C_1$-$C_4$-Alkylsulfonyl, $C_1$-$C_4$-Halogenalkylsulfonyl;

ix) Phenoxy oder Phenylthio, welche bis zu dreimal durch $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Halogen, Cyano oder Nitro substituiert sein können;

x) ein 5- oder 6-gliedriger heterocyclischer Rest mit einem oder zwei Heteroatomen, ausgewählt aus der Gruppe Sauerstoff, Schwefel und Stickstoff, der ein bis zwei Substituenten der folgenden Gruppen tragen kann: $C_1$-$C_3$-Alkyl, Halogen, $C_1$-$C_3$-Alkoxy oder $C_2$-$C_4$-Alkoxycarbonyl;

xi) Phenyl, welches eine bis drei der folgenden Gruppen tragen kann: $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro oder Cyano;

xii) eine Gruppe $OR^5$, wobei $R^5$ Wasserstoff, $C_1$-$C_4$-Alkyl,-carbonyl, $C_1$-$C_4$-Halogenalkylcarbonyl, $C_1$-$C_4$-Alk-

oxycarbonyl, $C_1$-$C_4$-Alkoxy-$C_2$-$C_4$-alkoxycarbonyl, $C_1$-$C_4$-Alkylcarbamoyl, $C_1$-$C_4$-Dialkylcarbamoyl, $C_1$-$C_4$-Alkylsulfonyl, $C_1$-$C_4$-Halogenalkylsulfonyl, Sulfamoyl, $C_1$-$C_4$-Alkylaminosulfonyl, $C_1$-$C_4$-Dialkylaminosulfonyl, Phenylsulfonyl, welches ein- bis dreimal durch $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, Halogen, Cyano oder Nitro substituiert sein kann, bedeutet;

xiii) eine Gruppe $NR^6R^7$, wobei $R^6$ Wasserstoff, $C_1$-$C_4$-Alkyl, Benzyl, $C_1$-$C_4$-Alkoxy oder zusammen mit $R^7$ C=S bedeutet und $R^7$ für Wasserstoff, $C_1$-$C_4$-Alkyl, $C_1$-$C_4$-Alkylcarbonyl, $C_1$-$C_4$-Halogenalkylcarbonyl, $C_1$-$C_4$-Alkoxycarbonyl, $C_1$-$C_4$-Alkoxy-$C_2$-$C_4$-alkoxycarbonyl, $C_1$-$C_4$-Alkylcarbamoyl, $C_1$-$C_4$-Dialkylcarbamoyl, $C_1$-$C_4$-Alkylsulfonyl, $C_1$-$C_4$-Halogenalkylsulfonyl, Sulfamoyl, $C_1$-$C_4$-Alkylaminosulfonyl, $C_1$-$C_4$-Dialkylaminosulfonyl, Phenylsulfonyl, welches ein- bis dreimal durch $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, Halogen, Cyano oder Nitro substituiert sein kann, steht;

ausgenommen 6-Amino-5-cyano-4-phenyl-pyridin-2,3-dicarbonsäureimid;
außerdem kann der Rest $R^3$ unabhängig von der jeweiligen Bedeutung von $R^2$ und $R^4$ die Bedeutung Nitro haben;
sowie deren landwirtschaftlich anwendbare Salze.

2. Pyridin-2,3-dicarbonsäureimide der Formel I gemäß Anspruch 1, in der die Substituenten folgende Bedeutung haben:
$R^1$
$C_1$-$C_6$-Alkyl, $C_3$-$C_6$-Alkenyl, $C_3$-$C_6$-Alkinyl, 1-Methylcyclopropyl, 1-Ethylcyclopropyl, 1-(Cyclopropyl)-$C_1$-$C_3$-alkyl oder $C_3$-$C_6$-Cycloalkyl;
zwei der drei Reste $R^2$, $R^3$ und $R^4$ unabhängig voneinander Wasserstoff, $C_1$-$C_4$-Alkyl, welches durch ein bis fünf Halogenatome substituiert sein kann, $C_1$-$C_4$-Alkoxy, Halogen oder Cyano und der dritte Rest wie in Anspruch 1 genannt.

3. Pyridin-2,3-dicarbonsäureimide der Formel I gemäß den Ansprüchen 1 und 2, in der $R^3$ für Nitro steht.

4. Herbizides Mittel, enthaltend ein Pyridin-2,3-dicarbonsäureimid der Formel I gemäß den Ansprüchen 1 bis 3 oder sein Salz sowie hierfür übliche Trägerstoffe.

5. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Pyridin-2,3-dicarbonsäureimids der Formel I gemäß den Ansprüchen 1 bis 3 behandelt.

6. Verfahren zur Herstellung von Pyridinderivaten der Formel I gemäß Anspruch 1, dadurch gekennzeichnet, daß man ein Pyridindicarbonsäureanhydrid der Formel II,

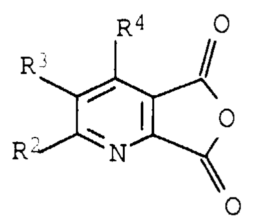

II

in der die Reste $R^2$ bis $R^4$ die in Anspruch 1 genannte Bedeutung haben, in an sich bekannter Weise in einem inerten organischen Lösungsmittel mit einem primären Amin III

$$H_2N\text{-}R^1 \qquad III$$

umsetzt und das so erhaltene Pyridindicarbonsäurehalbamid IV

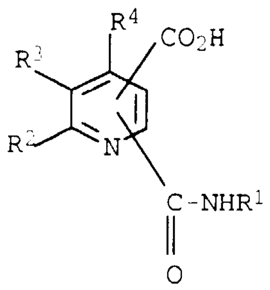

IV

mit wasserabspaltenden Mitteln zu I cyclisiert.

**7.** Verfahren zur Herstellung von nitrosubstituierten Pyridin-2,3-dicarbonsäureimiden der Formel Ia

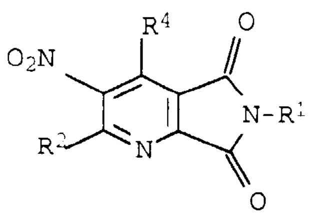

Ia

in der $R^2$ und $R^4$ die vorgenannte Bedeutung besitzen, dadurch gekennzeichnet, daß man ein entsprechendes Pyridin-2,3-dicarbonsäureimid der Formel Ib,

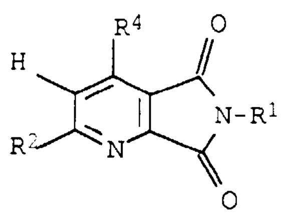

Ib

oder deren N-Oxid mit nitrierenden Agentien behandelt und gegebenenfalls die N-Oxidgruppe wieder abspaltet.

**8.** Verfahren zur Herstellung von nitrosubstituierten Pyridindicarbonsäureanhydriden der Formel IIa

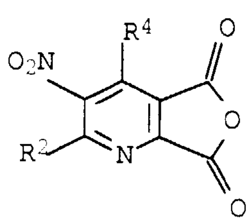

IIa

in der $R^2$ und $R^4$ die vorgenannte Bedeutung besitzen, dadurch gekennzeichnet, daß man Pyridindicarbonsäureanhydride der Formel IIb

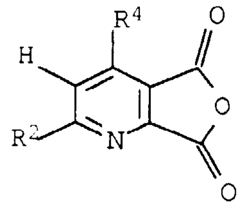

IIb

oder deren N-Oxid mit nitrierenden Agentien umsetzt und gegebenenfalls die N-Oxidgruppe wieder abspaltet.

**9.** Pyridin-2,3-dicarbonsäureanhydride der Formel IIa

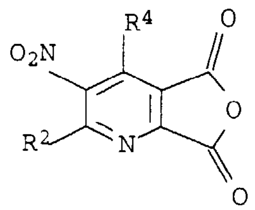

IIa

in der die Reste $R^2$ und $R^4$ die in Anspruch 1 genannte Bedeutung haben.

**10.** Pyridin-2,3-dicarbonsäureanhydride der Formel IIc

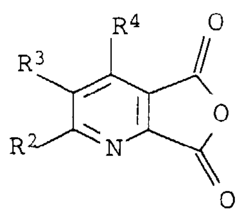

IIc

in der mindestens einer der Reste $R^2$, $R^3$ oder $R^4$ $NR^6R^7$ bedeutet und die übrigen Reste die in Anspruch 1 genannte Bedeutung haben.

**11.** Pyridin-2,3-dicarbonsäurediester der Formel Vb

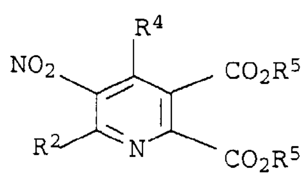

Vb

in der $R^2$ und $R^4$ die in Anspruch 1 genannte Bedeutung haben und $R^5$ einen $C_1$-$C_4$-Alkyl-, $C_3$-$C_5$-Alkenyl- oder $C_3$-$C_5$-Alkinylrest, gegebenenfalls substituiert durch Halogen, Phenyl oder $C_1$-$C_4$-Alkoxy darstellt, ausgenommen $R^2$ = Methyl, Halogen oder Amino, wenn $R^4$ Wasserstoff bedeutet.

**12.** Verfahren zur Herstellung von nitrosubstituierten Pyridindicarbonsäurediestern der Formel Vb gemäß Anspruch 11

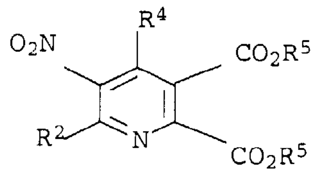

Vb

dadurch gekennzeichnet, daß man einen Pyridin-2,3-dicarbonsäureester der Formel Vb' oder deren N-Oxid

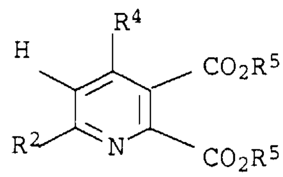

Vb'

in der $R^2$, $R^4$ und $R^5$ die vorgenannte Bedeutung besitzen, mit nitrierenden Agentien behandelt und gegebenenfalls anschließend die N-Oxidgruppe abspaltet.

**13.** Pyridin-2,3-dicarbonsäurediester der Formel Vd

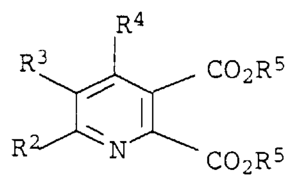

Vd

in der die Reste $R^2$, $R^3$ und/oder $R^4$ für eine Gruppe $NR^6R^7$ stehen, die übrigen Reste $R^2$, $R^3$ oder $R^4$ die in Anspruch 1 genannte Bedeutung haben und $R^5$ einen $C_1$-$C_4$-Alkyl-,
$C_3$-$C_5$-Alkenyl- oder $C_3$-$C_5$-Alkinylrest, gegebenenfalls substituiert durch Halogen, Phenyl oder $C_1$-$C_4$-Alkoxy darstellt, ausgenommen 5-Amino-6-methyl-pyridin-2,3-dicarbonsäurediethyl- und -dimethylester, 4-Amino-pyridin-2,3-dicarbonsäuredimethylester, 5,6-Diamino-pyridin-2,3-dicarbonsäurediethylester, 6-Amino-5-nitro-pyridin-2,3-dicarbonsäurediethylester, 5-Acetamino-6-methyl-pyridin-2,3-dicarbonsäurediethylester, 5-Methylamino-6-methyl-pyridin-2,3-dicarbonsäurediethylester, 4-Diethylamino-5-methyl-pyridin-2,3-dicarbonsäurediethyl- und -dimethylester, und 6-Diethylamino-5-methylpyridin-2,3-dicarbonsäurediethylester, 5-Acetylamino-pyridin-2,3-dicarbonsäurediethylester, 5-(N-Methyl-N-Acetylamino)-6-methylpyridin-2,3-dicarbonsäurediethylester, -5-Acetylamino-6-methyl-pyridin-2,3-dicarbonsäurediethylester, 4-Amino-5-cyano-6-(4-methylphenyl)sulfonylaminopyridin-2,3-dicarbonsäuredimethylester.

## Claims

**1.** A pyridine-2,3-dicarboximide of the general formula I

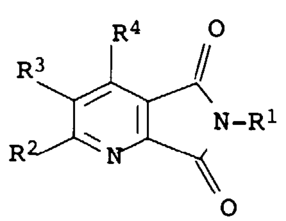

I

where
$R^1$ is hydrogen;
$C_1$-$C_4$-alkoxy;
$C_1$-$C_6$-alkyl which can carry one to three of the following groups: $C_1$-$C_4$-alkoxy, $C_1$-$C_4$-haloalkoxy, $C_1$-$C_4$-alkylthio, $C_1$-$C_4$-haloalkylthio, $C_1$-$C_4$-dialkylamino, $C_3$-$C_8$-cycloalkyl, halogen, excluding $C_1$-$C_4$-dialkylaminoethyl if one of the radicals $R^2$, $R^3$ or $R^4$ is amino or hydroxyl;
$C_3$-$C_8$-cycloalkyl which can carry one to three of the following groups: $C_1$-$C_6$-alkyl, $C_1$-$C_6$-haloalkyl, $C_1$-$C_4$-alkoxy, $C_1$-$C_4$-haloalkoxy, halogen or nitro;
$C_3$-$C_6$-alkenyl or $C_3$-$C_6$-alkynyl, which can be substituted up to three times by halogen, and
at least one radical $R^2$, $R^3$ or $R^4$ is a group $NR^6R^7$ and the other radicals $R^2$, $R^3$ and $R^4$ are defined as follows:

i) hydrogen;

ii) halogen or cyano;

iii) $C_1$-$C_6$-alkyl which can be substituted by one to five halogen atoms and/or one or two of the following radicals: $C_1$-$C_4$-alkoxy, $C_1$-$C_4$-haloalkoxy, $C_1$-$C_4$-alkylthio, $C_1$-$C_4$-haloalkylthio, $C_3$-$C_6$-cycloalkyl or cyano;

iv) benzyl which can be substituted up to three times by $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro;

v) $C_3$-$C_8$-cycloalkyl which can be substituted one to three times by $C_1$-$C_4$-alkyl or halogen;

vi) $C_2$-$C_6$-alkenyl which can be substituted up to three times by halogen and/or once by $C_1$-$C_3$-alkoxy or by phenyl which can carry one to three of the following groups: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro;

vii) $C_2$-$C_6$-alkynyl which can be substituted up to three times by halogen or $C_1$-$C_3$-alkoxy and/or once by phenyl which can carry one to three of the following groups: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro;

viii) $C_1$-$C_4$-alkoxy, $C_1$-$C_4$-alkylthio, $C_1$-$C_4$-haloalkoxy, $C_1$-$C_4$-haloalkylthio, $C_2$-$C_5$-alkenyloxy, $C_2$-$C_5$-alkynyloxy, $C_1$-$C_4$-alkylsulfinyl, $C_1$-$C_4$-alkylsulfonyl, $C_1$-$C_4$-haloalkylsulfonyl;

ix) phenoxy or phenylthio which can be substituted up to three times by $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, halogen, cyano or nitro;

x) a 5- or 6-membered heterocyclic radical having one or two heteroatoms selected from the group consisting of oxygen, sulfur and nitrogen, which can carry one or two of the following groups: $C_1$-$C_3$-alkyl, halogen, $C_1$-$C_3$-alkoxy or $C_2$-$C_4$-alkoxycarbonyl;

xi) phenyl which can carry one to three of the following groups: $C_1$-$C_6$-alkyl, $C_1$-$C_6$-haloalkyl, $C_1$-$C_6$-alkoxy, $C_1$-$C_6$-haloalkoxy, $C_1$-$C_6$-alkylthio, $C_1$-$C_6$-haloalkylthio, halogen, nitro or cyano;

xii) a group $OR^5$, where $R^5$ is hydrogen, $C_1$-$C_4$-alkylcarbonyl, $C_1$-$C_4$-haloalkylcarbonyl, $C_1$-$C_4$-alkoxycarbonyl, $C_1$-$C_4$-alkoxy- $C_2$-$C_4$-alkoxycarbonyl, $C_1$-$C_4$-alkylcarbamoyl, $C_1$-$C_4$-dialkylcarbamoyl, $C_1$-$C_4$-alkylsulfonyl, $C_1$-$C_4$-haloalkylsulfonyl, sulfamoyl, $C_1$-$C_4$-alkylaminosulfonyl, $C_1$-$C_4$-dialkylaminosulfonyl, phenylsulfonyl, which can be substituted one to three times by $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, halogen, cyano or nitro;

xiii) a group $NR^6R^7$, where $R^6$ is hydrogen, $C_1$-$C_4$-alkyl, benzyl, $C_1$-$C_4$-alkoxy or, together with $R^7$, is C=S and $R^7$ is hydrogen, $C_1$-$C_4$-alkyl, $C_1$-$C_4$-alkylcarbonyl, $C_1$-$C_4$-haloalkylcarbonyl, $C_1$-$C_4$-alkoxycarbonyl, $C_1$-$C_4$-alkoxy-$C_2$-$C_4$-alkoxycarbonyl, $C_1$-$C_4$-alkylcarbamoyl, $C_1$-$C_4$-dialkylcarbamoyl, $C_1$-$C_4$-alkylsulfonyl, $C_1$-$C_4$-haloalkylsulfonyl, sulfamoyl, $C_1$-$C_4$-alkylaminosulfonyl, $C_1$-$C_4$-dialkylaminosulfonyl, phenylsulfonyl, which can be substituted one to three times by $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, halogen, cyano or nitro;

excluding 6-amino-5-cyano-4-phenylpyridine-2,3-dicarboximide;

the radical $R^3$, independently of the respective meanings of $R^2$ and $R^4$, can additionally have the meaning nitro; or its agriculturally utilizable salts.

2. A pyridine-2,3-dicarboximide of the formula I as claimed in claim 1, where the substituents have the following meanings: $R^1$ is
$C_1$-$C_6$-alkyl, $C_3$-$C_6$-alkenyl, $C_3$-$C_6$-alkynyl, 1-methylcyclopropyl, 1-ethylcyclopropyl, 1-(cyclopropyl)-$C_1$-$C_3$-alkyl or $C_3$-$C_6$-cycloalkyl; two of the three radicals $R^2$, $R^3$ and $R^4$ independently of one another are hydrogen, $C_1$-$C_4$-alkyl which can be substituted by one to five halogen atoms, $C_1$-$C_4$-alkoxy, halogen or cyano and the third radical is as mentioned in claim 1.

3. A pyridine-2,3-dicarboximide of the formula I as claimed in claims 1 and 2, where $R^3$ is nitro.

4. A herbicidal composition, containing a pyridine-2,3-dicarboximide of the formula I as claimed in claims 1 to 3 or its salt, and carriers customary therefor.

5. A method of controlling undesired plant growth, which comprises treating the undesired plants and/or their habitat with a herbicidally effective amount of a pyridine-2,3-dicarboximide of the formula I as claimed in claims 1 to 3.

6. A process for preparing pyridine derivatives of the formula I as claimed in claim 1, which comprises reacting a pyridinedicarboxylic anhydride of the formula II

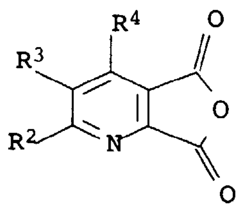

II

where the radicals $R^2$ to $R^4$ have the meanings mentioned in claim 1, with a primary amine III

$$H_2N\text{-}R^1 \qquad III$$

in a manner known per se in an inert organic solvent and cyclizing the resulting pyridinedicarboxylic acid hemiamide IV

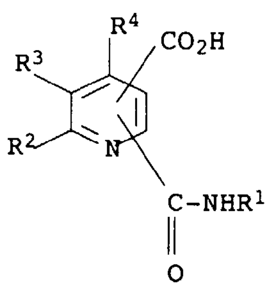

IV

with dehydrating agents to give I.

7. A process for preparing nitro-substituted pyridine-2,3-dicarboximides of the formula Ia

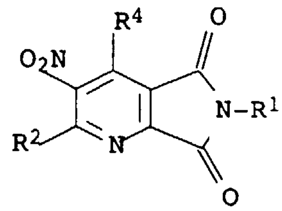

Ia

where $R^2$ and $R^4$ have the abovementioned meanings, which comprises treating a corresponding pyridine-2,3-dicarboximide of the formula Ib

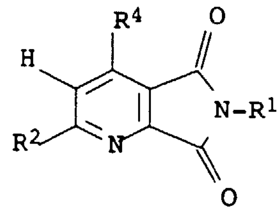

Ib

or its N-oxide with nitrating agents and, if appropriate, removing the N-oxide group again.

**8.** A process for preparing nitro-substituted pyridinedicarboxylic anhydrides of the formula IIa

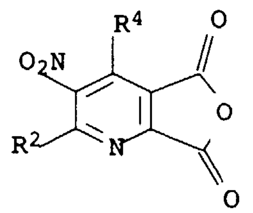

IIa

where $R^2$ and $R^4$ have the abovementioned meanings, which comprises reacting a pyridinedicarboxylic anhydride of the formula IIb

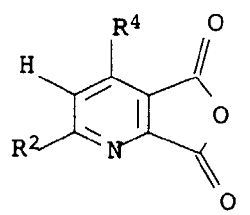

IIb

or its N-oxide with nitrating agents and, if appropriate, removing the N-oxide group again.

**9.** A pyridine-2,3-dicarboxylic anhydride of the formula IIa

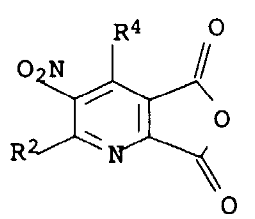

IIa

where the radicals $R^2$ and $R^4$ have the meanings mentioned in claim 1.

**10.** A pyridine-2,3-dicarboxylic anhydride of the formula IIc

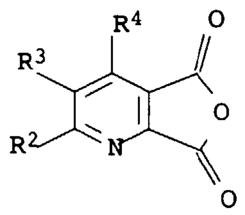

IIc

where at least one of the radicals $R^2$, $R^3$ or $R^4$ is $NR^6R^7$ and the other radicals have the meanings mentioned in claim 1.

**11.** A pyridine-2,3-dicarboxylic acid diester of the formula Vb

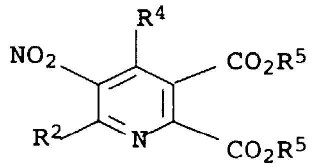

Vb

where $R^2$ and $R^4$ have the meanings mentioned in claim 1 and $R^5$ is a $C_1$-$C_4$-alkyl, $C_3$-$C_5$-alkenyl or $C_3$-$C_5$-alkynyl radical, which may be substituted by halogen, phenyl or $C_1$-$C_4$-alkoxy, excluding $R^2$ = methyl, halogen or amino if $R^4$ is hydrogen.

**12.** A process for preparing nitro-substituted pyridinedicarboxylic acid diesters of the formula Vb as claimed in claim 11

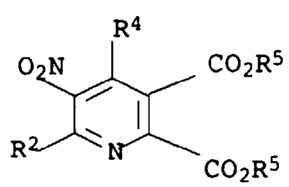

Vb

which comprises treating a pyridine-2,3-dicarboxylic acid ester of the formula Vb' or its N-oxide

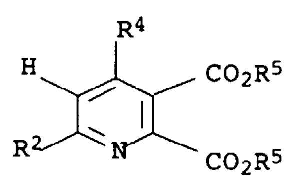

Vb'

where $R^2$, $R^4$ and $R^5$ have the abovementioned meanings, with nitrating agents and if appropriate then removing the N-oxide group.

**13.** A pyridine-2,3-dicarboxylic acid diester of the formula Vd

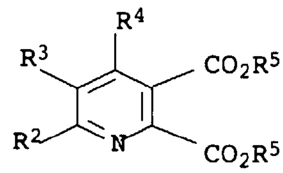

Vd

where the radicals $R^2$, $R^3$ and/or $R^4$ are an $NR^6R^7$ group, the remaining radicals $R^2$, $R^3$ or $R^4$ have the meanings mentioned in claim 1 and $R^5$ is a $C_1$-$C_4$-alkyl, $C_3$-$C_5$-alkenyl or $C_3$-$C_5$-alkynyl radical, which may be substituted by halogen, phenyl or $C_1$-$C_4$-alkoxy, excluding diethyl and dimethyl 5-amino-6-methylpyridine-2,3-dicarboxylate, dimethyl 4-aminopyridine-2,3-dicarboxylate, diethyl 5,6-diaminopyridine-2,3-dicarboxylate, diethyl 6-amino-5-nitropyridine-2,3-dicarboxylate, diethyl 5-acetamido-6-methylpyridine-2,3-dicarboxylate, diethyl 5-methylamino-6-methylpyridine-2,3-dicarboxylate, diethyl and dimethyl 4-diethylamino-5-methylpyridine-2,3-dicarboxylate and diethyl 6-diethylamino-5-methylpyridine-2,3-dicarboxylate, diethyl 5-acetylaminopyridine-2,3-dicarboxylate, diethyl 5-(N-methyl-N-acetylamino)-6-methylpyridine-2,3-dicarboxylate, diethyl 5-acetylamino-6-methylpyridine-2,3-dicarboxylate, dimethyl 4-amino-5-cyano-6-(4-methylphenyl)sulfonylaminopyridine-2,3-dicarboxylate.

## Revendications

**1.** Imides d'acides pyridine-2,3-dicarboxyliques, de formule générale I

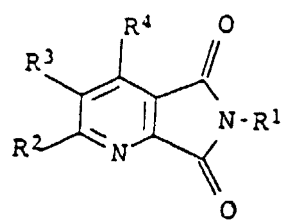

I

dans laquelle
$R^1$ représente
l'hydrogène ;
un groupe alcoxy en C1-C4 ;
un groupe alkyle en C1-C6 qui peut porter un à trois des substituants suivants : alcoxy en C1-C4, halogénoalcoxy en C1-C4, alkylthio en C1-C4, halogénoalkylthio en C1-C4, di-(alkyle en C1-C4)amine, cycloalkyle en C3-C8, halogéno, à l'exception de di-(alkyle en C1-C4)-aminoéthyle lorsqu'un des symboles $R^2$, $R^3$ ou $R^4$ représente un groupe amino ou hydroxy ;
un groupe cycloalkyle en C3-C8 qui peut porter un à trois des substituants suivants : alkyle en C1-C6, halogénoalkyle en C1-C6, alcoxy en C1-C4, halogénoalcoxy en C1-C4, halogéno ou nitro ;
un groupe alcényle en C3-C6 ou alcynyle en C3-C6 qui peut porter jusqu'à trois substituants halogéno et
au moins un des symboles $R^2$, $R^3$ ou $R^4$ représente un groupe $NR^6R^7$, les autres, parmi $R^2$, $R^3$ et $R^4$, ayant les définitions suivantes :

i) l'hydrogène ;

ii) un halogène ou un groupe cyano ;

iii) un groupe alkyle en C1-C6 qui peut porter un à cinq substituants halogéno et/ou un à deux des substituants suivants : alcoxy en C1-C4, halgoénoalcoxy en C1-C4, alkylthio en C1-C4, halogénoalkylthio en C1-C4, cycloalkyle en C3-C6 ou cyano ;

iv) un groupe benzyle qui peut porter jusqu'à trois substituants alkyle en C1-C4, halogénoalkyle en C1-C4, alcoxy en C1-C4, halogénoalcoxy en C1-C4, alkylthio en C1-C4, halogénoalkylthio en C1-C4, halogéno, cyano ou nitro ;

v) un groupe cycloalkyle en C3-C8 qui peut porter un à trois des substituants alkyle en C1-C4 ou halogéno ;

vi) un groupe alcényle en C2-C6 qui peut porter jusqu'à trois substituants halogéno et/ou un substituant alcoxy en C1-C3 ou phényle, lequel peut lui-même porter un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4, halogéno, cyano ou nitro ;

vii) un groupe alcynyle en C2-C6 qui peut porter jusqu'à trois substituants halogéno ou alcoxy en C1-C3 et/ou un substituant phényle, lequel peut lui-même porter un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4, halogéno, cyano ou nitro ;

viii) un groupe alcoxy en C1-C4, alkylthio en C1-C4, halogénoalcoxy en C1-C4, halogénoalkylthio en C1-C4, alcényloxy en C2-C5, alcynyloxy en C2-C5, alkylsulfinyle en C1-C4, alkylsulfonyle en C1-C4, halogénoalkylsulfonyle en C1-C4 ;

ix) un groupe phénoxy ou phénylthio qui peut porter jusqu'à trois substituants alkyle en C1-C4, halogénoalkyle en C1-C4, alcoxy en C1- C4, halogénoalcoxy en C1-C4, alkylthio en C1-C4, halogénoalkylthio en C1-C4, halogéno, cyano ou nitro ;

x) un radical hétérocyclique à cinq ou six chaînons contenant un ou deux hétéroatomes choisis parmi l'oxygène, le soufre et l'azote et qui peut porter un à deux substituants choisis parmi les groupes alkyle en C1-C3, les halogènes, les groupes alcoxy en C1-C3 ou (alcoxy en C2-C4)-carbonyle ;

xi) un groupe phényle qui peut porter un à trois des substituants suivants : alkyle en C1-C6, halogénoalkyle en C1-C6, alcoxy en C1-C6, halogénoalcoxy en C1-C6, alkylthio en C1-C6, halogénoalkylthio en C1-C6, halogéno, nitro ou cyano ;

xii) un groupe $OR^5$ dans lequel $R^5$ représente l'hydrogène, un groupe (alkyle en C1-C4)carbonyle, (halogénoalkyle en C1-C4)carbonyle, (alcoxy en C1-C4)carbonyle, (alcoxy en C1-C4)-(alcoxy en C2-C4)carbonyle, (alkyle en C1-C4)carbamoyle, di-(alkyle en C1-C4)carbamoyle, alkylsulfonyle en C1-C4, halogénoalkylsulfonyle en C1-C4, sulfamoyle, alkylaminosulfonyle en C1-C4, di-(alkyle en C1-C4)aminosulfonyle, phénylsulfonyle qui peut lui-même porter un à trois substituants alkyle en C1-C4, halogénoalkyle en C1-C4, alcoxy en C1-C4, halogénoalcoxy en C1-C4, alkylthio en C1-C4, halogéno, cyano ou nitro ;

xiii) un groupe $NR^6R^7$ dans lequel $R^6$ représente l'hydrogène, un groupe alkyle en C1-C4, benzyle, alcoxy en C1-C4 ou forme avec $R^7$ un groupe C=S, et $R^7$ représente l'hydrogène, un groupe alkyle en C1- C4, (alkyle en C1-C4)carbonyle, (halogénoalkyle en Cl-C4)carbonyle, (alcoxy en C1-C4)carbonyle, (alcoxy en C1-C4)-(alcoxy en C2-C4)carbonyle, (alkyle en C1-C4)carbamoyle, di-(alkyle en C1-C4)carbamoyle, alkylsulfonyle en C1-C4, halogénoalkylsulfonyle en C1-C4, sulfamoyle, alkylaminosulfonyle en C1-C4, di-(alkyle en C1-C4) aminosulfonyle, phénylsulfonyle qui peut lui-même porter un à trois substituants alkyle en C1-C4, halogénoalkyle en C1-C4, alcoxy en C1-C4, halogénoalcoxy en C1-C4, alkylthio en C1-C4, halogéno, cyano ou nitro ;

à l'exception de l'imide de l'acide 6-amino-5-cyano-4-phényl-pyridine-2,3-dicarboxylique ;
en outre, le symbole $R^3$, indépendamment des significations particulières de $R^2$ et $R^4$, peut représenter un groupe nitro ;
et leurs sels utilisables dans des applications agricoles.

**2.** Imides d'acides pyridine-2,3-dicarboxyliques de formule I selon la revendication 1, dans laquelle les symboles ont les significations suivantes : $R^1$
un groupe alkyle en C1-C6, alcényle en C3-C6, alcynyle en C3-C6, 1-méthylcyclopropyle, 1-éthylcyclopropyle, 1-(cyclopropyl)-alkyle en C1-C3 ou cycloalkyle en C3-C6 ;
deux des trois symboles $R^2$, $R^3$ et $R^4$ représentent chacun, indépendamment l'un de l'autre, l'hydrogène, un groupe alkyle en C1- C4 qui peut porter un à cinq atomes d'halogènes, un groupe alcoxy en C1-C4, un atome d'halogène ou un groupe cyano et le troisième de ces symboles a les significations indiquées dans la revendication 1.

**3.** Imides d'acides pyridine-2,3-dicarboxyliques de formule I selon les revendications 1 et 2 dans laquelle $R^3$ représente un groupe nitro.

4. Produit herbicide contenant un imide d'acide pyridine-2,3-dicarboxylique de formule I selon les revendications 1 à 3 ou l'un de ses sels et les véhicules usuels à cet effet.

5. Procédé pour combattre les croissances de végétaux indésirables, caractérisé par le fait que l'on traite les végétaux indésirables et/ou leur habitat par une quantité herbicide efficace d'un imide d'acide pyridine-2,3-dicarboxylique de formule I selon les revendications 1 à 3.

6. Procédé de préparation des dérivés de la pyridine répondant à la formule I de la revendication 1, caractérisé par le fait que l'on fait réagir un anhydride d'acide pyridine-dicarboxylique de formule II

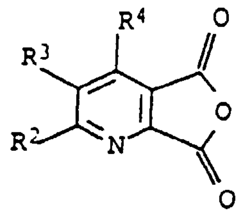

II

dans laquelle les symboles $R^2$ à $R^4$ ont des significations indiquées dans la revendication 1, de manière connue en soi, dans un solvant organique inerte, avec une amine primaire III

$$H_2N\text{-}R^1 \quad \text{III}$$

ce qui donne un hémiamide d'acide pyridinedicarboxylique IV

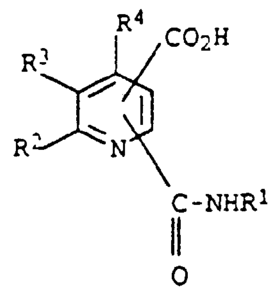

IV

qu'on cyclise en I à l'aide d'agents déshydratants.

7. Procédé de préparation des imides d'acides pyridine-2,3-dicarboxyliques à substituants nitro répondant à la formule Ia

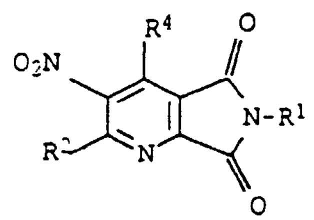

Ia

dans laquelle $R^2$ et $R^4$ ont les significations indiquées ci-dessus, caractérisé par le fait que l'on traite un imide d'acide pyridine-2,3-dicarboxylique correspondant, répondant à la formule Ib

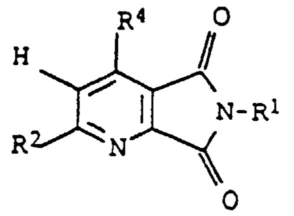

Ib

ou son N-oxyde par des agents nitrants et le cas échéant on élimine le N-oxyde.

**8.** Procédé de préparation des anhydrides d'acides pyridinedicarboxyliques à substituants nitro répondant à la formule IIa

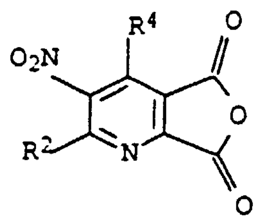

IIa

dans laquelle $R^2$ et $R^4$ ont les significations indiquées ci-dessus, caractérisé par le fait que l'on fait réagir des anhydrides d'acide pyridinedicarboxylique de formule IIb

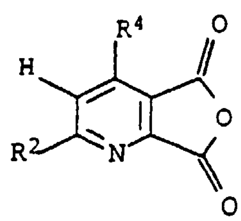

IIb

ou leur N-oxyde avec des agents nitrants et le cas échéant on élimine le groupe N-oxyde.

**9.** Anhydrides d'acides pyridine-2,3-dicarboxyliques de formule IIa

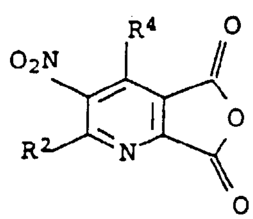

IIa

dans laquelle les symboles $R^2$ et $R^4$ ont les significations indiquées dans la revendication 1.

**10.** Anhydrides d'acides pyridine-2,3-dicarboxyliques de formule IIc

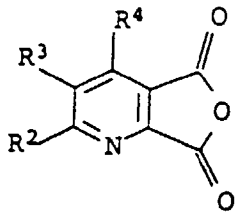

IIc

dans laquelle au moins un des symboles $R^2$, $R^3$ ou $R^4$ représente $NR^6R^7$ et les autres ont les significations indiquées dans la revendication 1.

**11.** Diesters d'acides pyridine-2,3-dicarboxyliques de formule Vb

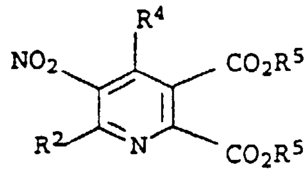

Vb

dans laquelle $R^2$ et $R^4$ ont les significations indiquées dans la revendication 1, et $R^5$ représente un groupe alkyle en C1-C4, alcényle en C3-C5 ou alcynyle en C3-C5 éventuellement substitué par des halogènes, un groupe phényle ou alcoxy en C1-C4, à l'exception de ceux pour lesquels $R^2$ représente un groupe méthyle, un halogène ou un groupe amino lorsque $R^4$ représente l'hydrogène.

**12.** Procédé de préparation des diesters d'acides pyridinedicarboxyliques à substituants nitro de formule Vb de la revendication 11

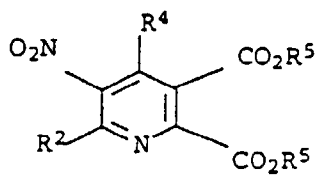

Vb

caractérisé par le fait que l'on traite un ester d'acide pyridine-2,3-dicarboxylique de formule Vb' ou son N-oxyde

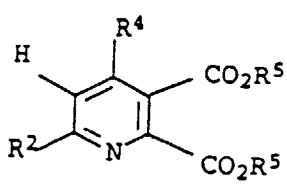

Vb'

$R^2$, $R^4$ et $R^5$ ayant les significations indiquées ci-dessus, par des agents nitrants et, le cas échéant, on élimine ensuite le groupe N-oxyde.

**13.** Diesters d'acides pyridine-2,3-dicarboxyliques de formule Vd

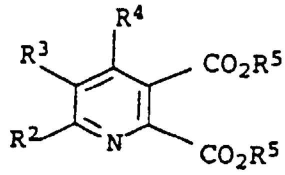

Vd

dans laquelle les symboles $R^2$, $R^3$ et/ou $R^4$ représentent un groupe $NR^6R^7$, les autres, parmi $R^2$, $R^3$ et $R^4$, ayant les significations indiquées dans la revendication 1, et $R^5$ représente un groupe alkyle en C1-C4, alcényle en C3-C5 ou alcynyle en C3-C5 portant éventuellement des substituants halogéno, phényle ou alcoxy en C1-C4, à l'exception du 5-amino-6-méthyl-pyridine-2,3-dicarboxylate de diéthyle et de diméthyle, du 4-aminopyridine-2,3-dicarboxylate de diméthyle, du 5,6-diamino-pyridine-2,3-dicarboxylate de diéthyle, du 6-amino-5-nitro-pyridine-2,3-dicarboxylate de diéthyle, du 5-acétamino-6-méthyl-pyridine-2,3-dicarboxylate de diéthyle, du 5-méthylamino-6-méthyl-pyridine-2,3-dicarboxylate de diéthyle, du 4-diéthylamino-5-méthyl-pyridine-2,3-dicarboxylate de diéthyle et de diméthyle, du 6-diéthylamino-5-méthyl-pyridine-2,3-dicarboxylate de diéthyle, du 5-acétylamino-pyridine-2,3-dicarboxylate de diéthyle, du 5-(N-méthyl-N-acétylamino)-6-méthyl-pyridine-2,3-dicarboxylate de diéthyle, du 5-acétylamino-6-méthyl-pyridine-2,3-dicarboxylate de diéthyle, du 4-amino-5-cyano-6-(4-méthylphényl)sulfonylaminopyridine-2,3-dicarboxylate de diméthyle.